(19) Europäisches Patentamt
European Patent Office
Office européen des brevets

(11) Veröffentlichungsnummer: **0 108 036**
**A1**

(12) # EUROPÄISCHE PATENTANMELDUNG

(21) Anmeldenummer: 83810429.7

(22) Anmeldetag: 26.09.83

(51) Int. Cl.[3]: C 07 D 493/04
A 61 K 31/37
//(C07D493/04, 311/00, 311/00)

(30) Priorität: 01.10.82 CH 5806/82

(43) Veröffentlichungstag der Anmeldung: 09.05.84 Patentblatt 84/19

(84) Benannte Vertragsstaaten: AT BE CH DE FR GB IT LI NL SE

(71) Anmelder: CIBA-GEIGY AG
Postfach
CH-4002 Basel(CH)

(72) Erfinder: Göschke, Richard, Dr.
Felixhäglistrasse 21
CH-4103 Bottmingen(CH)

(72) Erfinder: Wenk, Paul, Dr.
Quellenweg 7
CH-4123 Allschwil(CH)

(72) Erfinder: Ferrini, Pier Giorgio, Dr.
Im Rehwechsel 22
CH-4102 Binningen(CH)

(54) **Neue Benzopyranone.**

(57) 6-substituierte 4-oxo-4H-1-benzopyrane der formel

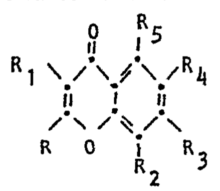

(I),

worin R freies, verestertes oder amidiertes Carboxy oder Tetrazolyl bedeutet, $R_1$ Wasserstoff oder einen aliphatischen Rest darstellt; $R_2$ Wasserstoff, einen aliphatischen Rest, Acyl oder gegebenenfalls verethertes oder verestertes Hydroxy bedeutet, $R_4$ eine Gruppe der Formel $-C(R_6)=C(R_7)-C(=O)-R_8$ darstellt, in der $R_6$ Wasserstoff oder einen gegebenenfalls durch Hydroxy substituierten aliphatischen Rest und $R_7$ Wasserstoff oder einen aliphatischen Rest bedeutet und $R_8$ gemeinsam mit einem der Reste $R_3$ und $R_5$ Oxy darstellen und der andere der Reste $R_3$ und $R_5$ Wasserstoff oder, im Falle von $R_5$, auch Hydroxy bedeutet, mit der Massgabe, dass R von Ethoxycarbonyl verschieden ist, wenn $R_4$ eine Gruppe der Formel $-C(R_6)=C(R_7)-C(=O)-R_8$ bedeutet, $R_8$ gemeinsam mit $R_3$ Oxy bedeutet, $R_1$, $R_2$, $R_5$ und $R_7$ Wasserstoff darstellen und $R_6$ Methyl ist, und Salze von Verbindungen der Formel I mit salzbildenden Eigenschaften, Verfahren zur Herstellung von Verbindungen der Formel I und ihrer Salze, ihre Verwendung, z.B. als Arzneimittelwirkstoffe, und pharmazeutische Präparate, die solche Verbindungen enthalten, sowie Formulierungsverfahren.

EP 0 108 036 A1

CIBA-GEIGY AG
Basel (Schweiz)

4-14113/+

Neue Benzopyranone

Die Erfindung betrifft neue Benzopyranonderivate, insbesondere 6-substituierte 4-Oxo-4H-1-benzopyrane der Formel

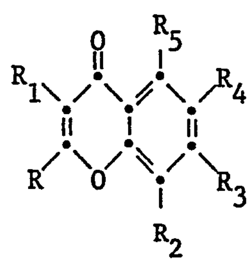

(I) ,

worin R freies, verestertes oder amidiertes Carboxy oder Tetrazolyl bedeutet, $R_1$ Wasserstoff oder einen aliphatischen Rest darstellt, $R_2$ Wasserstoff, einen aliphatischen Rest, Acyl oder gegebenenfalls verethertes oder verestertes Hydroxy bedeutet, $R_4$ eine Gruppe der Formel $-C(R_6)=C(R_7)-C(=O)-R_8$ darstellt, in der $R_6$ Wasserstoff oder einen gegebenenfalls durch Hydroxy substituierten aliphatischen Rest und $R_7$ Wasserstoff oder einen aliphatischen Rest bedeutet und $R_8$ gemeinsam mit einem der Reste $R_3$ und $R_5$ Oxy darstellen und der andere der Reste $R_3$ und $R_5$ Wasserstoff oder, im Falle von $R_5$, auch Hydroxy bedeutet, mit der Massgabe, dass R von Ethoxycarbonyl verschieden ist, wenn $R_4$ eine Gruppe der Formel $-C(R_6)=C(R_7)-C(=O)-R_8$ bedeutet, $R_8$ gemeinsam mit $R_3$ Oxy bedeutet, $R_1$, $R_2$, $R_5$ und $R_7$ Wasserstoff darstellen und $R_6$ Methyl ist, und Salze von Verbindungen der Formel I mit salzbildenden Eigenschaften, Verfahren zur Herstellung von Verbindungen der Formel I und ihrer

Salze, ihre Verwendung, z.B. als Arzneimittelwirkstoffe, und pharmazeutische Präparate, die solche Verbindungen enthalten, sowie Formulierungsverfahren.

Verestertes Carboxy ist beispielsweise mit einem gegebenenfalls, z.B. durch Hydroxy oder Acyloxy, substituierten Niederalkanol verestertes Carboxy, wie Niederalkoxycarbonyl, Hydroxyniederalkoxycarbonyl oder Niederalkanoyloxyniederalkoxycarbonyl.

Amidiertes Carboxy ist beispielsweise gegebenenfalls, z.B. ein- oder zweifach durch Niederalkyl, substituiertes Carbamoyl, wie Carbamoyl, N-Mono- oder N,N-Diniederalkylcarbamoyl, oder 1-Tetrazolylcarbonyl.

Tetrazolyl ist z.B. 5-Tetrazolyl.

Aliphatische Reste sind beispielsweise Niederalkyl-, ferner Niederalkenyl- oder in zweiter Linie Niederalkinylreste.

Acyl bzw. Acyl in Acyloxy ist beispielsweise Niederalkanoyl oder gegebenenfalls, z.B. durch Niederalkyl, Niederalkoxy und/oder Halogen, substituiertes Benzoyl.

Verethertes Hydroxy ist beispielsweise mit einem Niederalkanol verethertes Hydroxy, wie Niederalkoxy, ferner Niederalkenyloxy.

Verestertes Hydroxy ist beispielsweise mit einer Halogenwasserstoffsäure oder einer organischen Carbonsäure, wie Niederalkancarbonsäure, verestertes Hydroxy, wie Halogen oder Niederalkanoyloxy.

Durch Hydroxy substituierte aliphatische Reste sind beispielsweise Hydroxyniederalkylreste.

Die vor- und nachstehend verwendeten Allgemeindefinitionen haben in erster Linie die folgenden Bedeutungen:

Vor- und nachstehend sind unter "niederen" organischen Resten und Verbindungen vorzugsweise solche zu verstehen, die bis und mit 7, vor allem bis und mit 4 Kohlenstoffatome enthalten.

Niederalkoxy sowie Niederalkoxy in Niederalkoxycarbonyl ist z.B. Methoxy, Ethoxy, Propyloxy, Isopropyloxy, n-Butyloxy, Isobutyloxy, sec-Butyloxy oder tert-Butyloxy, ferner ein Pentyloxy-, Hexyloxy- oder Heptyloxyrest.

Hydroxyniederalkoxycarbonyl ist z.B. 2-Hydroxyethoxy-, 2- oder 3-Hydroxypropyloxy-, oder 2,3-Dihydroxypropyloxycarbonyl.

Niederalkanoyloxy und Niederalkanoyloxy in Niederalkanoyloxyniederalkoxycarbonyl ist z.B. Acetyl-, Propionyl-, Butyryl-, Isobutyryl- oder Pivaloyloxy.

N-Mononiederalkylcarbamoyl ist z.B. N-Methyl-, N-Ethyl-, N-Propyl-, N-Isopropyl- oder N-Butylcarbamoyl, und N,N-Diniederalkylcarbamoyl ist z.B. N,N-Dimethyl- oder N,N-Diethylcarbamoyl.

Niederalkyl ist z.B. Methyl, Ethyl, Propyl, Isopropyl, n-Butyl, Isobutyl, sec-Butyl, tert-Butyl, ferner ein Hexyl- oder Heptylrest.

Niederalkenyl ist z.B. 1- oder 2-Propenyl, 1-, 2- oder 3-Butenyl

oder 1-Methyl-prop-2-enyl, ferner 1,3-Butadienyl. Niederalkinyl ist z.B. Ethinyl, 2-Propinyl, 1-, 2- oder 3-Butinyl.

Hydroxyniederalkyl ist z.B. 2-Hydroxyethyl, 2- oder 3-Hydroxypropyl, 1,2-Dihydroxyethyl oder 2,3-Dihydroxypropyl.

Niederalkenyloxy ist z.B. Ethenyloxy, 1,2- oder 1,3-Propenyloxy oder 1,2-, 1,3- oder 1,4-Buten-2-yloxy.

Halogen ist z.B. Halogen bis und mit Atomnummer 35, wie Fluor, Chlor oder Brom, ferner Iod.

Niederalkanoyl ist z.B. Formyl, Acetyl, Propionyl, Butyryl, Isobutyryl oder Pivaloyl.

Salze der neuen Verbindungen sind insbesondere pharmazeutisch verwendbare Salze, beispielsweise pharmazeutisch verwendbare Salze von Verbindungen der Formel I, worin R Carboxy bzw. Tetrazolyl bedeutet, mit Basen, ferner Oxoniumsalze mit Säuren. Als Salze mit Basen kommen beispielsweise Alkalimetall- oder Erdalkalimetallsalze, wie Natrium-, Kalium-, Calcium- oder Magnesiumsalze, ferner Ammoniumsalze mit Ammoniak oder organischen Aminen, wie Mono- oder Diniederalkylaminen, z.B. Methylamin oder Dimethylamin, oder Mono-, Di- oder Triniederalkanolaminen, z.B. Ethanolamin, Diethanolamin oder Triethanolamin, in Betracht. Oxoniumsalze mit Säuren sind beispielsweise Hydrohalogenide, wie Hydrochloride oder -iodide, oder

Perchlorate. Umfasst sind ferner für pharmazeutische Verwendung ungeeignete Salze, da diese beispielsweise für die Isolierung bzw. Reinigung freier erfindungsgemässer Verbindungen sowie derer pharmazeutisch verwendbarer Salze verwendet werden können.

Die Verbindungen der allgemeinen Formel I und ihre pharmazeutisch verwendbaren Salze weisen wertvolle pharmakologische Eigenschaften auf. So besitzen sie beispielsweise eine ausgeprägte antiallergische Wirksamkeit, die sich z.B. an der Ratte in Dosen von etwa 1 bis etwa 100 mg/kg bei oraler Verabreichung bzw. im Dosisbereich von etwa 0,01 bis etwa 10 mg/kg bei i.v. Applikation im passiven kutanen Anaphylaxie-Test nachweisen lässt, der analog der von Goose und Blair, Immunology, Bd. 16, S. 749 (1969), beschriebenen Methode durchgeführt wird. Die passive kutane Anaphylaxie wird dabei nach dem von Ovary, Progr. Allergy, Bd. 5, S. 459 (1958), beschriebenen Verfahren erzeugt. Die erfindungsgemässen Verbindungen der Formel I sind deshalb z.B. bei der Bekämpfung allergischer Reaktionen, z.B. in der Behandlung und Prophylaxe von allergischen Erkrankungen, wie Asthma, sowohl extrinsic als auch intrinsic Asthma, oder anderen allergischen Erkrankungen, wie Heufieber, Konjunctivitis, Uritcaria und Ekzeme, verwendbar.

Die Erfindung betrifft beispielsweise Verbindungen der Formel I, worin R Carboxy, Niederalkoxycarbonyl, Hydroxyniederalkoxycarbonyl, Niederalkanoyloxyniederalkoxycarbonyl, Carbamoyl, N-Mono- oder N-Diniederalkylcarbamoyl, 1-Tetrazolyl-carbonyl oder 5-Tetrazolyl bedeutet, $R_1$ Wasserstoff, Niederalkyl oder Niederalkenyl ist, $R_2$ Wasserstoff, Niederalkyl, Niederalkenyl, Niederalkanoyl, gegebenenfalls durch Niederalkyl, Niederalkoxy und/oder Halogen substituiertes Benzoyl, Hydroxy, Niederalkoxy, Niederalkenyloxy, Halogen oder Niederalkanoyloxy darstellt, $R_4$ eine Gruppe der Formel

$-C(R_6)=C(R_7)-C(=O)-R_8$ bedeutet, in der $R_6$ Wasserstoff, Niederalkyl, Niederalkenyl oder Hydroxyniederalkyl und $R_7$ Wasserstoff, Niederalkyl oder Niederalkenyl bedeutet, und $R_8$ gemeinsam mit einem der Reste $R_3$ und $R_5$ Oxy bedeutet und der andere der Reste $R_3$ und $R_5$ Wasserstoff oder, im Falle von $R_5$, auch Hydroxy bedeutet, mit der Massgabe, dass R von Ethoxycarbonyl verschieden ist, wenn $R_4$ eine Gruppe der Formel $-C(R_6)=C(R_7)-C(=O)-R_8$ bedeutet, $R_8$ gemeinsam mit $R_3$ Oxy bedeutet und $R_1$, $R_2$, $R_5$ und $R_7$ Wasserstoff darstellen und $R_6$ Methyl ist, und Salze, insbesondere pharmazeutisch verwendbare Salze vorstehender Verbindungen mit salzbildenden Eigenschaften.

Die Erfindung betrifft in erster Linie Verbindungen der Formel I, worin R Carboxy, Niederalkoxycarbonyl oder Niederalkanoyloxyniederalkoxycarbonyl bedeutet, $R_1$ Wasserstoff oder in zweiter Linie Niederalkyl darstellt, $R_2$ Wasserstoff, Niederalkyl, Niederalkenyl, Hydroxy, Niederalkoxy, Niederalkanoyloxy oder Halogen bedeutet, $R_4$ eine Gruppe der Formel $-C(R_6)=C(R_7)-C(=O)-R_8$ darstellt, in der $R_6$ und $R_7$ unabhängig voneinander Wasserstoff oder Niederalkyl bedeuten und $R_8$ gemeinsam mit einem der Reste $R_3$ und $R_5$ Oxy bedeutet und der andere der Reste $R_3$ und $R_5$ Wasserstoff ist, mit der Massgabe, dass R von Ethoxycarbonyl verschieden ist, wenn $R_4$ eine Gruppe der Formel $-C(R_6)=C(R_7)-C(=O)-R_8$ bedeutet, $R_8$ gemeinsam mit $R_3$ Oxy bedeutet, $R_1$, $R_2$, $R_5$ und $R_7$ Wasserstoff darstellen und $R_6$ Methyl bedeutet, und Salze, insbesondere pharmazeutisch verwendbare Salze vorstehender Verbindungen mit salzbildenden Eigenschaften.

Die Erfindung betrifft vor allem Verbindungen der Formel I, worin R Carboxy oder Niederalkoxycarbonyl mit bis und mit 4 C-Atomen im Niederalkylteil, wie Methoxy- oder Ethoxycarbonyl, bedeutet, $R_1$ für Wasser-

stoff steht, $R_2$ Wasserstoff, Niederalkyl mit bis und mit 4 C-Atomen, wie Methyl oder Propyl,oder Niederalkenyl mit bis und mit 4 C-Atomen, wie Allyl, bedeutet, $R_4$ eine Gruppe der Formel $-C(R_6)=C(R_7)-C(=O)-R_8$ darstellt, in der $R_6$ Niederalkyl mit bis und mit 4 C-Atomen, wie Methyl, darstellt, $R_7$ Wasserstoff bedeutet und einer der Reste $R_3$ und $R_5$ gemeinsam mit $R_8$ Oxy darstellt und der andere Wasserstoff bedeutet, und Salze, insbesondere pharmazeutisch verwendbare Salze solcher Verbindungen mit salzbildenden Eigenschaften.

Die Verbindung betrifft in allererster Linie Verbindungen der Formel I, worin R Carboxy bedeutet, $R_1$ für Wasserstoff steht, $R_2$ Niederalkyl mit bis und mit 4 C-Atomen, in erster Linie Propyl, oder Niederalkenyl mit bis und mit 4 C-Atomen, in erster Linie Allyl, bedeutet, $R_4$ eine Gruppe der Formel $-C(R_6)=C(R_7)-C(=O)-R_8$ darstellt, in der $R_6$ Niederalkyl mit bis und mit 4 C-Atomen, in erster Linie Methyl, bedeutet, $R_7$ Wasserstoff bedeutet und einer der Reste $R_3$ und $R_5$ gemeinsam mit $R_8$ Oxy darstellt und der andere Wasserstoff bedeutet, und Salze, insbesondere pharmazeutisch verwendbare Salze solcher Verbindungen mit salzbildenden Eigenschaften.

Die Efindung betrifft namentlich die in den Beispielen genannten Verbindungen der Formel I sowie die dort beschriebenen Verfahren zu ihrer Herstellung.

Die neuen Verbindungen der Formel I können in an sich bekannter Weise hergestellt werden, z.B. indem man Verbindungen der Formel

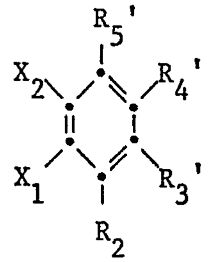

(IIa) ,

worin $X_1$ Hydroxy oder reaktionsfähiges verestertes Hydroxy bedeutet, $X_2$ eine Gruppe der Formel $-CO-CH(R_1)-CO-R$ bedeutet oder worin $X_1$ eine Gruppe der Formel $-O-C(R)=C(R_1)-CO-R_9$ und $R_9$ eine geeignete Abgangsgruppe bedeutet und $X_2$ Wasserstoff ist und $R_3'$, $R_4'$ bzw. $R_5'$ jeweils die Bedeutungen von $R_3$, $R_4$ bzw. $R_5$ haben, oder worin jeweils $X_1$ eine Gruppe der Formel $-O-C(R)=C(R_1)-CO-X_1'$ bedeutet und $X_1'$ mit $X_2$ eine gemeinsame Bindung bedeuten und einer der Reste $R_3'$ und $R_5'$ eine Gruppe der Formel $-O-CO-CH(R_7)-CO-R_6$ und der andere die Bedeutung von $R_3$ bzw. $R_5$ hat und $R_4'$ Wasserstoff ist, oder einer der Reste $R_3'$ und $R_5'$ Hydroxy oder reaktionsfähiges verestertes Hydroxy bedeutet und der andere die Bedeutung von $R_3$ bzw. $R_5$ hat und $R_4'$ eine Gruppe der Formel $-C(R_6)=C(R_7)-CO-R_9$ und $R_9$ eine geeignete Abgangsgruppe bedeutet, oder worin einer der Reste $R_3'$ und $R_5'$ eine Gruppe der Formel $-O-CO-C(R_7)=P(X_3)_3$ und $X_3$ einen aliphatischen oder aromatischen Rest oder Niederalkoxy bzw. gegebenenfalls substituiertes Phenoxy bedeutet und der andere die Bedeutung von $R_3$ bzw. $R_5$ hat und $R_4'$ eine Gruppe der Formel $-CO-R_6$ bedeutet, oder Salze von solchen Verbindungen cyclisiert, erforderlichenfalls ein verfahrensgemäss erhältliches Isomerengemisch in die Isomeren auftrennt und das gewünschte Isomere isoliert und gewünschtenfalls eine so erhältliche Verbindung in eine andere Verbindung der Formel I umwandelt und/oder eine so erhältliche freie Verbindung in ein Salz oder ein so erhältliches Salz in die freie Verbindung oder ein anderes Salz überführt.

Die folgenden Reste kommen beispielsweise als geeignete Abgangsgruppe $R_9$ in Betracht: Hydroxy, Halogen, wie Chlor, Niederalkoxy, wie Methoxy oder Ethoxy, gegebenenfalls substituiertes Phenoxy, Acyloxy, wie Niederalkanoyloxy oder gegebenenfalls substituiertes

Benzoyloxy.

Reaktionsfähiges verestertes Hydroxy ist z.B. in erster Linie Halogen, ferner Acyloxy, wie Niederalkanoyloxy, oder Sulfonyloxy, wie Niederalkan- bzw. gegebenenfalls, z.B. durch Niederalkyl oder Halogen, substituiertes Phenylsulfonyloxy.

Ein aliphatischer Rest $X_3$ ist beispielsweise ein Niederalkylrest, z.B. tert-Butyl. Ein aromatischer Rest $X_3$ ist beispielsweise gegebenenfalls substituiertes Phenyl.

Salze von Verbindungen der Formel IIa können z.B. für den Fall, dass R Carboxy ist, Salze mit Basen sein, wie Alkalimetall-, z.B. Natriumsalze derselben, ferner können Oxoniumsalze mit starken Säuren, beispielsweise Mineralsäuren, wie Halogenwasserstoff- oder Halogensauerstoffsäuren, gebildet werden, wie Oxoniumiodide oder -perchlorate.

Die erfindungsgemässe Umsetzung kann in üblicher Weise, insbesondere in der aus der Literatur für analoge Reaktionen bekannten Weise erfolgen, vorzugsweise in Gegenwart eines Katalysators und unter wasserfreien Bedingungen, und erforderlichenfalls unter Kühlen und Erwärmen, beispielsweise bei einer Reaktionstemperatur von etwa 50°C bis etwa 200°C, sowie in einem inerten Lösungsmittel und/oder unter Inertgas, z.B. Stickstoff. Als Lösungsmittel eignen sich insbesondere polare Lösungsmittel, beispielsweise Amine, wie Pyridin, Amide, wie Dimethylformamid, oder auch unpolare Lösungsmittel, z.B. Ether, wie Dioxan oder Tetrahydrofuran. Geeignete katalytische Mittel sind vorzugsweise Säuren, wie Mineralsäuren, z.B. Schwefelsäure, Polyphosphorsäure, Halogenwasserstoff-, z.B. Chlorwasserstoffsäure, oder organische Carbonsäuren, z.B. Niederalkancarbonsäure, wie Essigsäure, und/oder deren Anhydride, wie Acetanhydrid, oder Sulfonsäuren wie gegebenenfalls z.B. durch

Halogen, substituierte Niederalkan- oder gegebenenfalls, z.B. durch Halogen oder Niederalkyl,substituierte Phenylsulfonsäuren. Ferner kann erforderlichenfalls die Verwendung von Lewissäuren, wie Aluminiumchlorid, angezeigt sein, insbesondere bei solchen Verbindungen der Formel (IIa), worin $X_1$ eine Gruppe der Formel $-O-C(R)=C(R_1)-CO-R_9$ bedeutet oder worin $R_3'$ bzw. $R_5'$ eine Gruppe der Formel $-O-CO-CH(R_7)-CO-R_6$ bedeutet. Die Cyclisierung kann auch durch Erhitzen und/oder unter neutralen oder basischen Bedingungen erfolgen. Bedeutet $X_1$ oder $R_3'$ bzw. $R_5'$ beispielsweise Halogen, wird die entsprechende Cyclisierung in Gegenwart einer starken Base durchgeführt. Als geeignete Basen kommen beispielsweise Alkalimetallhydroxide, z.B. Natronlauge, Alkalimetallniederalkanolate, z.B. Natriummethylat, gegebenenfalls cyclische tertiäre Stickstoffbasen, z.B. Triethylamin, Ethyl-diisopropylamin, Piperidin, Collidin, 1,5-Diaza-bicyclo[4.3.0]non-5-en (DBN) oder 1,8-Diaza-bicyclo[5.4.0]undec-7-en (DBU), ferner Lithiumdiniederalkylamide, z.B. Lithium-diethyl- oder Lithium-diisopropyl-amid, verwendet werden.

Die Ausgangsverbindungen der Formel IIa, worin $X_1$ eine Gruppe der Formel $-O-C(R)=C(R_1)-C(=O)-R_9$ bedeutet, in der $R_9$ eine geeignete Abgangsgruppe darstellt, $X_2$ Wasserstoff ist und $R_3'$, $R_4'$ bzw. $R_5'$ die Bedeutungen von $R_3$, $R_4$ bzw. $R_5$ haben, können nach an sich bekannten Verfahren hergestellt werden. So gelangt man in einer bevorzugten Ausführungsform zu diesen Verbindungen, indem man Verbindungen der Formel

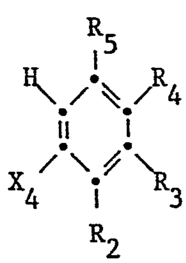

(IIb) ,

worin $X_4$ eine Gruppe der Formel OM bedeutet, in der M Wasserstoff oder ein Metall-, wie Alkalimetallkation darstellt, mit Verbindungen

der Formel

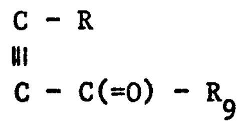

(IIc)

oder deren Salzen mit Basen umsetzt. Bei der Reaktion von Verbindungen der Formel IIb mit Verbindungen der Formel IIc werden bevorzugt solche Verbindungen der Formel IIa erhalten, worin $R_1$ Wasserstoff bedeutet.

Zur Herstellung von Ausgangsmaterial der Formel IIa, worin $X_1$ Hydroxy oder reaktionsfähiges verestertes Hydroxy bedeutet, $X_2$ eine Gruppe der Formel $-CO-CH(R_1)-CO-R$ bedeutet, geht man beispielsweise von Verbindungen der Formel

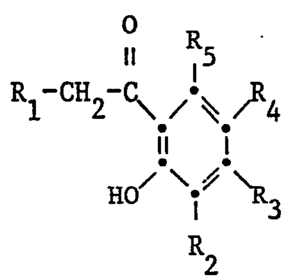

(IId)

aus und setzt diese z.B. mit einer Verbindung der Formel $R-CO-R_9$ (IIe) in Gegenwart einer Base um.

Die Reaktionen werden bevorzugt in Gegenwart einer Base, beispielsweise eines Alkalimetalles, wie feinverteiltes Natrium, Alkalimetallhydrides, -amides, -alkanolates, -carbonates oder -hydroxides, wie Natriumhydrid, -amid, -methylat, Kaliumcarbonat oder Natriumhydroxid, durchgeführt.

Zu Ausgangsstoffen der Formel IIa, worin $X_1$ eine Gruppe der Formel $-O-C(R)=C(R_1)-C(=O)-X_1'$ bedeutet und $X_1'$ gemeinsam mit $X_2$ eine Bildung bildet, einer der Reste $R_3'$ und $R_5'$ Hydroxy oder reaktions-

fähiges verestertes Hydroxy darstellt und der andere die Bedeutung von $R_3$ bzw. $R_5$ hat und der Rest $R_4'$ für die Gruppe der Formel $-C(R_6)=C(R_7)-C(=O)-R_9$ steht und $R_9$ eine geeignete Abgangsgruppe bedeutet, gelangt man, indem man beispielsweise Verbindungen der Formel

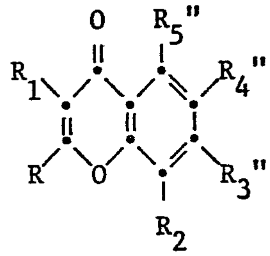

(IIe)

worin einer der Reste $R_3''$ und $R_5''$ Hydroxy oder reaktionsfähiges verestertes Hydroxy bedeutet und der andere die Bedeutung von $R_3$ bzw. $R_5$ hat und $R_4''$ eine Gruppe der Formel $-C(=O)-R_6$ darstellt mit Verbindungen der Formel

$$(X_3)_3P=C(R_7)-C(=O)-R_9 \qquad (IIf) \quad ,$$

oder Salzen derselben umsetzt, wobei die so erhältlichen Verbindungen der Formel IIa vorteilhaft <u>in situ</u> gebildet werden und unter den Reaktionsbedingungen direkt zur Verbindung der Formel I weiterreagieren können.

Zur Herstellung von Ausgangsmaterial der Formel (IIa), worin $X_1$ eine Gruppe der Formel $-O-C(R)=C(R_1)-CO-X_1'$ bedeutet, $X_1'$ mit $X_2$ eine gemeinsame Bindung bedeuten, einer der Reste $R_3'$ und $R_5'$ eine Gruppe der Formel $-O-CO-C(R_7)=P(X_3)_3$ bedeutet, der andere der Reste $R_3'$ bzw. $R_5'$ die Bedeutung von $R_3$ bzw. $R_5$ hat und $R_4'$ eine Gruppe der Formel $-CO-R_6$ bedeutet, geht man z.B. von Verbindungen der Formel

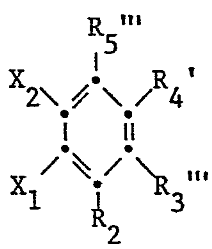

(IIg)

aus, worin $R_3'''$ bzw. $R_5'''$ für eine Gruppe der Formel $-O-CO-CH(R_7)-Hal$ steht und Hal für Halogen, wie Brom, steht und setzt diese in Gegenwart einer Base mit Verbindungen der Formel $P(X_3)_3$ um.

Die neuen Verbindungen der Formel I können ferner hergestellt werden, z.B. indem man aus einer Verbindung der Formel

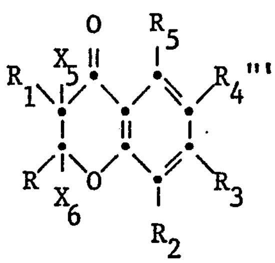

(III) ,

worin $R_4'''$ eine Gruppe der Formel $-C(R_6)(X_5')-C(R_7)(X_6')-CO-R_8$ bedeutet, die Variablen $X_5$, $X_6$, $X_5'$ und $X_6'$ jeweils Wasserstoff oder Halogen bedeuten oder eines der Variablenpaare $X_5$ und $X_6$ bzw. $X_5'$ und $X_6'$ für eine gemeinsame Bindung steht und die Variablen des anderen Variablenpaares jeweils Wasserstoff oder Halogen bedeuten bzw. eine der Variablen des anderen Variablenpaares Wasserstoff ist und die andere eine geeignete abspaltbare Gruppe bedeutet, oder ein Salz davon unter Bildung einer oder zwei zusätzlicher Bindungen eine Verbindung der Formel $X_5' - X_6'$ und/oder $X_5 - X_6$ abspaltet, erforderlichenfalls ein Verfahren gemäss erhältliches Isomerengemisch in die Isomerengemisch in die Isomeren auftrennt und das gewünschte Isomere isoliert und gewünschtenfalls eine so erhältliche Verbindung in eine andere Verbindung der Formel I umwandelt und/oder eine so erhältliche freie Verbindung in ein Salz oder ein so erhältliches Salz in die freie Verbindung oder ein anderes Salz überführt.

Als abspaltbare Gruppen $X_5$ bzw. $X_6$ und/oder $X_5'$ bzw. $X_6'$ eignen sich beispielsweise Hydroxy, Halogen, Sulfonyloxy, wie gegebenenfalls, z.B.

durch Halogen, substituiertes Niederalkan- oder gegebenenfalls, z.B. durch Halogen oder Niederalkyl, substituiertes Phenylsulfonyloxy, Diniederalkylsulfonium, wie Dimethylsulfonium, Triniederalkylammonium, wie Trimethylammonium, oder N-Oxido-diniederalkylamino, wie Dimethyl-N-oxido-amino. Ferner kommen beispielsweise Niederalkanoyloxy, Niederalkyl-thiocarbonyloxy (Thionoester-Typ), Niederalkoxy-thiocarbonyl-oxy (Thiocarbonatester-Typ) oder Niederalkylthio-thiocarbonyloxy-(Xanthatester-Typ) in Frage.

Salze von Verbindungen der Formel III sind, sofern z.B. R Carboxy bedeutet, Salze mit Basen, wie Alkalimetall-, z.B. Natriumsalze derselben, ferner Oxoniumsalze mit starken Säuren, beispielsweise Mineralsäuren, wie Halogenwasserstoff- oder Halogensauerstoffsäuren, z.B. entsprechende Iodide oder Perchlorate.

Die Abspaltung $X_5$ - $X_6$ bzw. $X_5'$ - $X_6'$ aus Verbindungen der Formel III, worin $X_5$ und $X_6$ bzw. $X_5'$ und $X_6'$ Wasserstoff bedeuten, kann beispielsweise durch Oxidation in Gegenwart eines milden Oxidations- bzw. Dehydrierungsmittels, wie eines Chinons, z.B. Tetrachlor-p-benzochinon oder 2,3-Dichlor-5,6-dicyano-p-benzochinon, Phenanthren-9,10-chinon, einer Selenverbindung, z.B. Diphenylselenium-bis-trifluoracetat oder Selendioxid, Selen, Bleitetraacetat oder Triphenylmethylperchlorat, durchgeführt werden. Eine weitere Möglichkeit besteht beispielsweise darin, dass man Verbindungen der Formel III, worin $X_5$ und $X_6$ bzw. $X_5'$ und $X_6'$ Wasserstoff bedeuten, durch Behandeln mit einem Halogenierungsmittel, beispielsweise mit einem N-Halogensuccinimid, zu Verbindungen der Formel III, worin einer der Reste $X_5$ und $X_6$ bzw. $X_5'$ und $X_6'$ Wasserstoff und der andere Halogen darstellt, halogeniert und anschliessend beispielsweise unter Verwendung einer Base, wie eines tertiären Amins, z.B. Dimethylphenylamin oder Pyridin, dehydrohalogeniert.

Die Abspaltung von Verbindungen der Formel $X_5 - X_6$ bzw. $X_5' - X_6'$, in der einer der Reste $X_5$ und $X_6$ bzw. $X_5'$ und $X_6'$ Wasserstoff und der andere Hydroxy bedeutet, aus einer Verbindung der Formel III oder ein Salz davon erfolgt in üblicher, insbesondere in der aus der Literatur für analoge Reaktionen bekannten Weise. Die Umsetzung wird erforderlichenfalls unter Kühlen oder Erwärmen und/oder in Gegenwart eines katalytischen Mittels durchgeführt. Als Katalysatoren eignen sich vorzugsweise Säuren. Säuren sind beispielsweise anorganische Säuren, wie Mineralsäuren, z.B. Schwefelsäure, Halogenwasserstoffsäuren oder Polyphosphorsäure, oder organische Säuren, wie Niederalkancarbonsäuren, z.B. Essigsäure. Die Eliminierung von Verbindungen der Formel $X_5-X_6$ bzw. $X_5'-X_6'$, wobei einer der Reste $X_5$ bzw. $X_5'$ Wasserstoff ist und der andere Halogen, Sulfonyloxy, Diniederalkylsulfonium, Triniederalkylammonium oder N-Oxido-diniederalkylamino wird in erster Linie in Gegenwart einer Base durchgeführt. Als Basen kommen beispielsweise Alkalimetallhydroxide, wie Natrium- oder Kaliumhydroxid, Alkalimetallcarbonate, wie Natrium- oder Kaliumcarbonat, Alkalimetallalkoholate, wie Natriummethylat, oder Amine, wie primäre, sekundäre oder tertiäre Amine, z.B. tert-Butyl-, Dimethyl-, Triethylamin oder Pyridin, in Frage. Dabei arbeitet man erforderlichenfalls in einem inerten Lösungsmittel, beispielsweise einem Alkohol, wie Niederalkanol, z.B. Methanol oder Ethanol, in einem Ether, wie Dioxan oder Tetrahydrofuran, und/oder unter Inertgas, wie Stickstoff.

Die Abspaltung von Wasser $X_5 - X_6$ bzw. $X_5' - X_6'$ aus Verbindungen der Formel III erfolgt dementsprechend bevorzugt in Gegenwart einer Säure, z.B. einer Mineralsäure, wie Schwefelsäure oder Halogenwasserstoffsäure, die Eliminierung von Halogenwasserstoff, z.B. Chlorwasserstoff, und Sulfonsäuren, z.B. p-Toluolsulfonsäure, unter basischen Bedingungen, beispielsweise in Gegenwart eines tertiären Amins, wie Triethylamin oder Pyridin, und die Eliminierung von tertiären Aminen, z.B. Trimethylamin, Hydroxylaminen, z.B. Dimethylhydroxylamin, oder Niederalkancarbonsäuren, z.B. Essigsäure, unter Erhitzen. Die Abspaltung von Verbindungen der Formeln $X_5 - X_6$ und/oder $X_5' - X_6'$, in denen einer der Reste $X_5$ und $X_6$ bzw. $X_5'$ und $X_6'$ Wasserstoff und der andere Niederalkanoyloxy, Niederalkylthiocarbonyl-oxy, Niederalkoxy-thiocarbonyl-oxy oder Niederalkylthio-thiocarbonyl-oxy bedeutet, wird, z.B. in Anlehnung an die Chugaev-Reaktion, thermisch durch Pyrolyse, beispielsweise in einem Temperaturbereich von etwa 100° bis etwa 250°C durchgeführt.

Bedeuten $X_5$ und $X_6$ und/oder $X_5'$ und $X_6'$ jeweils Halogen, wie Chlor, Brom oder Iod, kann ein oder zwei Moleküle Halogen unter Bildung einer oder zweier zusätzlicher Bindungen z.B. unter reduktiven Bedingungen wie in Gegenwart von unedlen Metallen, z.B. Zink oder Magnesium, von metallorganischen Reduktionssystemen, wie Alkalimetallsalzen von Dimethylsulfoxid, von Alkalimetalliodiden, z.B. Natriumiodid in Aceton, oder von Alkalimetallsulfiden, wie Natriumsulfid, in Gegenwart von Phasentransferkatalysatoren, eliminiert werden.

Die Ausgangsstoffe der Formel III oder ihre Salze werden nach an sich bekannten Verfahren überwiegend in situ gebildet und unter den Reaktionsbedingungen ohne Isolierung weiter zu den gewünschten Verbindungen der Formel I umgewandelt. Dabei kann die Abspaltung von $X_5 - X_6$ gleichzeitig mit der Cyclisierung oder im Anschluss an die vorgelagerte Cyclisierung erfolgen.

So gelangt man beispielsweise zu Verbindungen der Formel III, in der $X_5$ Wasserstoff ist und $X_6$ Wasserstoff oder eine geeignete abspaltbare Gruppe bedeutet und $R_4'''$ eine Gruppe der Formel $-C(R_6)(X_5')-C(R_7)(X_6')-C(=O)-R_8$ darstellt und $X_5'$ und $X_6'$ gemeinsam eine zusätzliche Bildung bilden, indem man Verbindungen der Formel

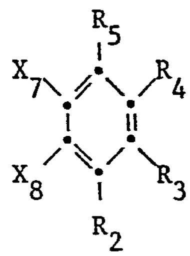

(IIIa) ,

worin $X_7$ eine Gruppe der Formel $-C(=O)-C(R_1)=C(X_6'')-R$ oder eine

tautomerere Form davon, in der $X_6$" Hydroxy, Halogen, Sulfonyloxy, Acyloxy, Niederalkylthio, gegebenenfalls substituiertes und/ oder N-oxidiertes Amino darstellt, und $X_8$ Hydroxy bedeutet, erforderlichenfalls in Gegenwart eines katalytischen Mittels und unter inerten Verfahrensbedingungen, beispielsweise unter Kühlen und Erwärmen, z.B. in einer Temperaturbereich von etwa -10° bis etwa +100°C, und in einem inerten Lösungsmittel, cyclisiert, gegebenenfalls in einer so erhältlichen Verbindung der Formel III, worin $X_6$ Niederalkylthio oder gegebenenfalls substituiertes Amino bedeutet, diese Gruppe in üblicher Weise erschöpfend alkyliert und unter den Reaktionsbedingungen erfindungsgemäss weiter umsetzt. Als katalytische Mittel werden bevorzugt Säuren verwendet, beispielsweise Mineralsäuren, wie Schwefelsäure, Chlorsulfonsäure, Polyphosphorsäure oder Halogenwasserstoffsäuren, z.B. Chlorwasserstoff-, Brom- oder Jodwasserstoffsäure, die gegebenenfalls als Gemische mit einem Carbonsäureanhydrid, wie Acetanhydrid, oder einer Carbonsäure, wie Essigsäure, eingesetzt werden.

Ferner kann man Verbindungen der Formel IIIa, worin $X_7$ eine Gruppe der Formel $-C(=O)-CH_2-R_1$ und $X_8$ eine Gruppe der Formel $-O-C(=O)-R$ bedeutet, unter den vorstehend aufgeführten Bedingungen, zu Verbindungen der Formel IIIa, worin $X''_6$ Hydroxy bedeutet, umsetzen oder gegebenenfalls direkt <u>in situ,</u> cyclisieren und die so gebildeten Verbindungen der Formel III ohne Isolierung erfindungsgemäss in Verbindungen der Formel I überführen.

Verbindungen der Formel III', worin $X_5$ und $X_6$ insbesondere Wasserstoff bedeuten, können hergestellt werden, indem man Verbindungen der Formel

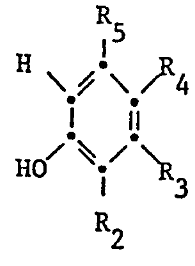

(IIIb),

mit Verbindungen der Formel

$$HOOC-C(R_1)=CH-R \qquad (IIIc)$$

in Gegenwart einer Säure <u>in situ</u> zu der entsprechenden Verbindung der Formel III cyclisiert. Dabei kommen als Säure insbesondere Mineralsäuren, wie Polyphosphorsäure oder Halogenwasserstoffsäuren, z.B. Fluorwasserstoffsäure, in Frage.

Zu Verbindungen der Formel III, worin $X_5$ und $X_6$ eine gemeinsame Bindung bilden und $R_4'''$ eine Gruppe der Formel $-C(R_6)(X_5')-C(R_7)(X_6')-C(=O)-R_8$ bedeutet, in der einer der Reste $X_5'$ und $X_6'$ Wasserstoff und der andere Wasserstoff oder eine geeignete abspaltbare Gruppe darstellt, gelangt man beispielsweise, in dem man Verbindungen der Formel

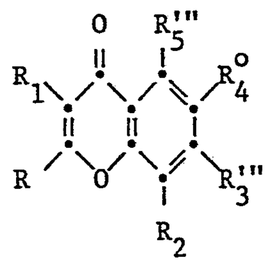

(IIId),

worin einer der Reste $R_3'''$ und $R_5'''$ Hydroxy bedeutet und der andere die Bedeutung von $R_3$ bzw. $R_5$ hat und $R_4^o$ eine Gruppe der Formel $-C(R_6)=C(R_7)-C(=O)-R_9$ darstellt, in der $R_9$ eine geeignete Abgangsgruppe bedeutet, eine Verbindung der Formel $X_5'-X_6'$ addiert und die so erhaltenen Verbindungen zu den entsprechenden Verbindungen der Formel III cyclisiert.

Die folgenden Reste kommen beispielsweise als geeignete Abgangsgruppe $R_9$ in Betracht: Hydroxy, Halogen, wie Chlor, Niederalkoxy, wie Methoxy oder Ethoxy, gegebenenfalls substituiertes Phenoxy, Acyloxy, wie Niederalkanoyloxy oder gegebenenfalls substituiertes Benzoyloxy.

In einer bevorzugten Ausführungsform des vorstehenden Verfahrens gelangt man, ausgehend von Verbindungen der Formeln IIIa und IIIb, zu entsprechenden Verbindungen der Formel III, welche unter den beschriebenen Reaktionsbedingungen zu den erfindungsgemässen Verbindungen umgesetzt werden.

Verbindungen der Formel I oder Salze davon kann man ferner herstellen, indem man in einer Verbindung der Formel

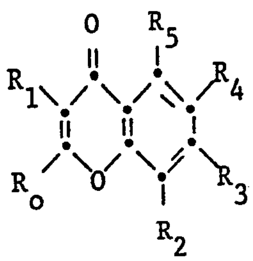

(IV),

worin $R_o$ einen in R überführbaren Rest bedeutet, $R_o$ in den Rest R überführt und gewünschtenfalls eine so erhältliche Verbindung in eine andere Verbindung der Formel I umwandelt und/oder eine so erhältliche freie Verbindung in ein Salz oder ein so erhältliches Salz in die freie Verbindung oder ein anderes Salz überführt.

Einen Substituenten $R_o$ kann man beispielsweise durch Solvolyse, wie Hydrolyse, Alkoholyse, Amino- oder Ammonolyse, in den Rest R überführen, wobei als Solvolysemittel Wasser, ein entsprechender Alkohol, ein geeignetes Aminderivat oder Ammoniak verwendet wird. Derartige Reste $R_o$ sind beispielsweise von freiem, verestertem oder amidiertem Carboxy bzw. Tetrazolyl R verschiedene, funktionell abgewandelte Carboxygruppen, wie die Cyanogruppe, anhydridisierte Carboxygruppen, beispielsweise Halogen-, z.B. Chlorcarbonyl, mit einem Alkohol, z.B. Niederalkanol, veretherte und/oder mit einer Mineralsäure, z.B. Halogenwasserstoffsäure, anhydridisierte Orthosäuregruppierungen, beispielsweise Triniederalkoxy-, Trihalogen- oder Niederalkoxyhalogenmethyl, wie Trimethoxy-, Trichlor- oder Dimethoxychlormethyl,

oder mit einer Carbonsäure, z.B. Niederalkancarbonsäure oder Kohlensäurehalbester, anhydridisiertes Carboxy, beispielsweise Niederalkanoyloxycarbonyl, wie Acetyloxycarbonyl oder Ethoxycarbonyloxycarbonyl. Ferner kommen als derartige funktionell abgewandelte Carboxygruppen Amidinogruppen, beispielsweise Amidino- oder Niederalkylamidinogruppen, Iminoestergruppierungen, beispielsweise Imid- oder Amidhalogenidgruppen, z.B. Iminochlor- oder Aminodichlormethyl, oder freie bzw. veresterte oder amidierte Thiocarbonylgruppen, beispielsweise Niederalkylthiocarbonylgruppen, z.B. Ethylthiocarbonyl, oder Thiocarbonyl, in Betracht.

Derartige, von freiem, verestertem oder amidiertem Carboxyl R verschiedene Gruppen können beispielsweise hydrolytisch in freies Carboxy R überführt werden, während z.B. die Cyanogruppe, Iminoester- bzw. Amidinogruppen zu amidiertem und Orthoestergruppierungen zu verestertem Carboxy R hydrolysiert werden. Durch Alkoholyse bzw. Amino- oder Ammonolyse kann man beispielsweise anhydridisiertes Carboxy in verestertes bzw. amidiertes Carboxy überführen.

Solvolysereaktionen werden in üblicher Weise mit Hilfe eines Solvolysemittels, erforderlichenfalls in Gegenwart eines katalytischen Mittels, in einem Lösungs- oder Verdünnungsmittel, unter Kühlen oder Erwärmen und/oder unter Inertgas durchgeführt. Als katalytische Mittel kommen beispielsweise bei der Hydrolyse saure oder basische Mittel in Frage, wie Mineralsäuren, z.B. Schwefelsäure oder Chlorwasserstoffsäure, Alkalimetallhydroxide oder -carbonate, z.B. Natrium- oder Kaliumhydroxid, Natrium- oder Kaliumcarbonat, in Frage. Bei der Alkoholyse bzw. Aminolyse können als katalytische Mittel beispielsweise Basen, wie die vorstehend genannten, tertiäre Amine, wie Triniederalkylamine, z.B. Triethylamin, oder Pyridin, verwendet werden.

Weitere in R überführbare Gruppen sind oxidativ in den Rest R überführbare Gruppen $R_o$, beispielsweise gegebenenfalls hydratisierte

Formylgruppen. Diese können vorteilhaft im Verlaufe der Oxidationsreaktion, z.B. aus der Acylgruppe einer gegebenenfalls α,β-ungesättigten oder α,β-dihydroxylierten oder araliphatischen Carbonsäure oder aus einer gegebenenfalls veresterten Hydroxymethylgruppe, in situ gebildet oder aus einem ihrer funktionellen Derivate, z.B. einem ihrer Acetale, Acylale oder Imine, in Freiheit gesetzt werden. Acylgruppen von gegebenenfalls α,β-ungesättigten oder α,β-dihydroxylierten Carbonsäuren sind beispielsweise Alkanoylgruppen, wie Niederalkanoyl, z.B. Acetyl, Acylgruppen von α,β-ungesättigten aliphatischen Mono- oder Dicarbonsäuren, z.B. Acryloyl, Crotonyl oder die Acyclgruppe der gegebenenfalls funktionell abgewandelten Fumar- oder Maleinsäure, Acylgruppen von α,β-ungesättigten araliphatischen Carbonsäuren, z.B. gegebenenfalls substituiertes Cinnamoyl, oder Acylgruppen von aliphatischen α,β-Dihydroxydicarbonsäuren, wie der Weinsäure, oder monofunktioneller Carboxyderivate, wie Estern oder Amiden, derselben. Verestertes Hydroxymethyl ist beispielsweise mit einer Mineralsäure, wie einer Halogenwasserstoffsäure, z.B. mit Chlor- oder Bromwasserstoffsäure, oder mit einer Carbonsäure, z.B. mit Essigsäure oder der gegebenenfalls substituierten Benzoesäure, verestertes Hydroxymethyl. Acetalisiertes Formyl ist beispielsweise mit Niederalkanolen oder einem Niederalkandiol acetalisiertes Formyl, wie Dimethoxy-, Diethoxy- oder Ethylendioxyacetyl. Acylalisiertes Formyl ist beispielsweise mit einer Mineralsäure acylalisiert und bedeutet z.B. Dihalogen-, wie Dichlormethyl.Weitere oxidativ in die Carboxygruppe überführbare Reste $R_o$ sind z.B. gegebenenfalls substituierte, wie in 5-Stellung eine acetalisierte Formylgruppe, wie Diethoxymethyl, aufweisende 2-Furoylgruppen. Ferner kann eine Alkyl-, wie Niederalkylgruppe, zu Carboxy oxidiert werden.

Weitere oxidativ in R überführbare Gruppen $R_o$ sind veretherte Hydroxymethylgruppen, wie Niederalkoxymethyl, die zu verestertem Carboxy R oxidiert werden können.

Die Oxidation erfolgt jeweils in üblicher Weise, beispielsweise durch Umsetzung mit einem Oxidationsmittel. Als Oxidationsmittel eignen sich beispielsweise Sauerstoff, Ozon, Peroxide, wie Wasserstoffperoxid, oder Peroxide von organischen Carbonsäuren, wie Trifluorperessigsäure oder m-Chlorperbenzoesäure, oxidierende Verbindungen von Uebergangsmetallen, insbesondere solche mit Elementen der I, VI., VII. oder VIII. Nebengruppe des Periodensystems, wie Kupferverbindungen, z.B. Kuperchromit, wie Silververbindungen, z.B. Silber(I)-oxid oder Silberpicolinat, wie Chromverbindungen, z.B. Chromylchlorid, chromtrioxid, Alkalimetallchromate oder -dichlromate, wie Kaliumdichromat, wie Manganverbindungen, z.B. Mangandioxid oder Alkalimetallpermanganate, oder wie Halogen-Sauerstoff-Verbindungen, z.B. Alkalimetalliodate oder -periodate, ferner wie Halogen, z.B. Brom oder Chlor, Halogen-Sauerstoff-Verbindungen, z.B. Alkalimetallhypochlorite, -iodate, -periodate oder Periodsäure, Stickstoffsäuren oder durch Anhydride, z.B. Salpetersäure oder entsprechende Anhydride konzentrierte Schwefelsäure. Erforderlichenfalls können auch Gemische von Oxidationsmitteln eingesetzt werden.

Dabei wird häufig in Gegenwart von Basen, wie Alkalimetallhydroxiden oder -carbonaten, z.B. Natriumhydroxid oder -carbonat, oder wie Aminen, z.B. cyclische Amine, z.B. Pyridin, oder Niederalkylamine, z.B. Triethylamin, oder von Protonsäuren, wie Mineralsäuren, z.B. Schwefelsäure oder Halogenwasserstoffsäure, oder organischen Carbonsäuren, wie Niederalkancarbonsäuren, z.B. Essigsäure, und gegebenenfalls unter Kühlen oder Erwärmen gearbeitet.

Als Lösungs- bzw. Verdünnungsmittel kommen beispielsweise Wasser, Ether, wie Dioxan oder Ethylenglykoldiethylether, Ketone, wie Aceton, Alkohole, wie die Niederalkanole Methanol oder Ethanol, Amide, wie Dimethylformamid, Carbonsäuren, wie die Niederalkancarbonsäure Essigsäure, oder Gemische derselben in Frage.

Die Oxidation von Formyl oder Hydroxymethyl zu Carboxy und von verethertem Hydroxymethyl zu verestertem Carboxy wird z.B. vorteilhaft mit Kaliumpermanganat in wässrigem Pyridin oder Aceton bei Raumtemperatur vorgenommen. Acetalisiertes Formyl und Iminomethylgruppen werden vorzugsweise sauer oxidiert, z.B. mit Natriumchromat oder Kaliumdichromat in Schwefelsäure. Acylgruppen von α,β-dihydroxylierten aliphatischen Carbonsäuren, wie der Acylrest der Weinsäure, werden vorteilhaft mit Periodsäure oxidiert. Niederalkyl kann beispielsweise unter Verwendung von Selendioxid, z.B. durch Erhitzen in wässrigem Dioxan, oxidiert werden.

In Tetrazolyl R überführbar ist beispielsweise die Imidazidgruppe, welche z.B. <u>in situ</u> durch Umsetzung der Cyanogruppe $R_o$ mit einem Azid, beispielsweise einem Alkalimetallazid, wie Natrium- oder Ammoniumazid, erforderlichenfalls unter Erwärmen, vorzugsweise in Gegenwart eines Halogenids, wie Ammonium- oder Aluminiumchlorid, erhalten werden kann und z.B. in einem inerten Lösungsmittel unter den Reaktionsbedingungen in eine Tetrazolylgruppe R übergeführt werden kann.

Die Ausgangsstoffe der Formel IV oder deren Salze werden nach an sich bekannten Verfahren hergestellt. Beispielsweise können sie hergestellt werden, indem man Verbindungen der Formel

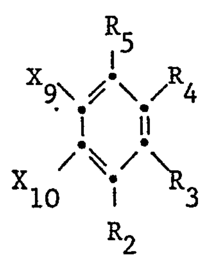

(IVa) ,

worin $X_9$ Wasserstoff ist und $X_{10}$ eine Gruppe der Formel $-O-C(R_o)=C(R_1)-C(=O)-R_9$ bedeutet, in der $R_9$ eine geeignete Abgangsgruppe darstellt, <u>in situ</u> in Gegenwart einer Protonsäure und/oder Lewissäure cyclisiert, vorzugsweise wie vorstehend für die Cyclisierung von Verbindungen der Formeln IIa, IIIa bzw. IIId angegeben.

Die folgenden Reste kommen beispielsweise als geeignete Abgangsgruppen $R_9$ in Betracht: Hydroxy, Halogen, wie Chlor, Niederalkoxy, wie Methoxy oder Ethoxy, gegebenenfalls substituiertes Phenoxy, Acyloxy, wie Niederalkanoyloxy oder gegebenenfalls substituiertes Benzoyloxy.

Ein weiterer Weg zur Herstellung von Verbindungen der Formel IV besteht in der Kondensation von Verbindungen der Formel

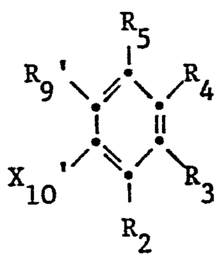

(IVb) ,

worin $X_9'$ eine Gruppe der Formel $-C(=O)-CH(R_1)-C(=O)-R_o$ und $X_{10}'$ Hydroxy bedeutet. Diese Umsetzung erfolgt analog der zu Verbindungen der Formel III führenden Weise (ausgehend von Verbindungen der Formel IIIa), woran sich eine weitere Umsetzung in Gegenwart einer Säure anschliesst.

In einer weiteren Verfahrensweise kann man zu Verbindungen der Formel I gelangen, indem man beispielsweise in einer Verbindung der Formel

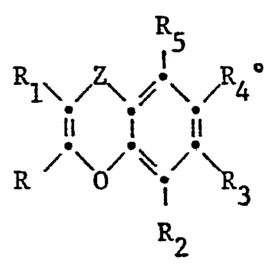

(V)

oder einem Salz oder Tautomeren davon, worin $R_4°$ für eine Gruppe

der Formel $-C(R_6)=C(R_7)-Z'-R_8$ steht und eine der Gruppen Z' und Z für eine in die Carbonylgruppe überführbare Gruppe steht und die andere die Carbonylgruppe oder eine in die Carbonylgruppe überführbare Gruppe bedeutet, Z' und/oder Z in die Carbonylgruppe überführt und gewünschtenfalls eine so erhältliche Verbindung in eine andere Verbindung der Formel I überführt und/oder eine so erhältliche freie Verbindung in ein Salz oder ein so erhältliches Salz in die freie Verbindung oder in ein anderes Salz überführt.

Eine in die Carbonylgruppe überführbare Gruppe Z' bzw. Z ist beispielsweise die Methylen- oder Hydroxymethylengruppe, Dihalogenmethylen, Thiocarbonyl oder Iminomethylen.

Die Methylen- bzw. Hydroxymethylengruppe wird oxidativ in an sich bekannter Weise in die Carbonylgruppe überführt, wobei in Gegenwart eines üblichen Oxidationsmittels gearbeitet wird. Derartige Oxidationsmittel sind beispielsweise Chromatverbindungen, wie Natriumchromat, Kaliumdichromat, tert-Butylchromat, oder Chromtrioxid (z.B. in Form des Pyridiniumkomplexes), oder Manganverbindungen, wie Kaliumpermanganat.

Die Dihalogenmethylen- bzw. die Iminomethylengruppe Z bzw. Z' wird z.B. durch Hydrolyse in die Carbonylgruppe überführt, beispielsweise durch Behandeln mit Wasser und/oder einem Niederalkanol. Ebenso kann die Thiocarbonylgruppe z.B. durch (oxidative) Hydrolyse, gegebenenfalls in Gegenwart einer Verbindung der Gruppe Ib, IIb oder IIIb des Periodensystems, durchgeführt werden. Geeignete, die Hydrolyse unterstützende Mittel sind z.B. Quecksilber-, Thallium- oder Silberverbindungen, wie Quecksilber(II)acetat oder -chlorid, Thallium(III)trifluoracetat oder Silberoxid.

Die Umsetzungen können in an sich bekannter Weise durchgeführt werden, z.B. in Ab- oder üblicherweise in Anwesenheit eines geeigneten Lösungs- oder Verdünnungsmittels oder eines Gemisches derselben, wobei unter Kühlen, bei Raumtemperatur oder unter Erwärmen, z.B. in einem Temperaturbereich von etwa 0° bis etwa 200°C, falls erforderlich unter Druck und/oder in einer Inertgasatmosphäre gearbeitet werden kann.

Die Ausgangsverbindungen der Formel V können in an sich bekannter Weise hergestellt werden. So geht man beispielsweise zur Herstellung von Verbindungen der Formel V, worin Z für die Methylen- oder Hydroxymethylengruppe steht, von einer Verbindung der Formel

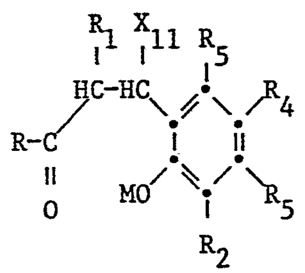

(Va) ,

einem Salz oder Isomeren davon aus, worin M für Wasserstoff oder ein Metall-, wie Alkalimetallkation steht, $X_{11}$ Wasserstoff oder gegebenenfalls, z.B. durch eine Acylgruppe, wie Acetyl, geschützte Hydroxygruppe bedeutet, und cyclisiert diese z.B. in Gegenwart einer die Cyclisierung unterstützenden starken Säure. Zur Herstellung von Verbindungen der Formel (V), worin Z eine Dihalogenmethylengruppe bedeutet, behandelt man beispielsweise eine Verbindung der Formel IV mit einem Halogenierungsmittel, wie Thionyl- oder Oxalylchlorid und führt anschliessend die Ueberführung von

$R_o$ in R in der angegebenen Weise durch. Ebenfalls von Verbindungen der Formel IV ausgehend gelangt man z.B. zu Verbindungen der Formel V, worin Z Thiocarbonyl bedeutet, indem man die entsprechenden Verbindungen der Formel IV z.B. mit $P_4S_{10}$ umsetzt und anschliessend in der angegebenen Weise $R_o$ in R überführt.

Eine erfindungsgemäss erhältliche Verbindung der allgemeinen Formel I kann in an sich bekannter Weise in eine andere Verbindung der allgemeinen Formel I umgewandelt werden.

So kann man freie, veresterte und amidierte Carboxygruppen R ineinander, beispielsweise eine freie Carboxygruppe in üblicher Weise in eine veresterte Carboxylgruppe R, überführen, vorzugsweise durch Umsetzung mit einem entsprechenden Alkohol oder einem reaktionsfähigen Derivat, wie einem Carbon-, Phosphorig-, Schweflig- oder Kohlensäureester, z.B. einem Niederalkancarbonsäureester, Triniederalkylphosphit, Diniederalkylsulfit oder dem Pyrocarbonat, oder einem Mineralsäure- oder Sulfonsäureester, z.B. dem Chlor- oder Bromwasserstoffsäure- oder Schwefelsäure-, Benzolsulfonsäure-, Toluolsulfonsäure- oder Methansulfonsäureester, des entsprechenden Alkohols oder einem davon abgeleiteten Olefin.

Die Umsetzung mit dem entsprechenden Alkohol erfolgt vorteilhaft in Gegenwart eines sauren Katalysators, wie einer Protonensäure, z.B. von Chlor- oder Bromwasserstoff-, Schwefel-, Phosphor-, Bor-, Benzolsulfon- und/oder Toluolsulfonsäure, oder einer Lewissäure, z.B. von Bortrifluorid-Etherat, in einem inerten Lösungsmittel, insbesondere einem Ueberschuss des eingesetzten Alkohols und erforderlichenfalls

in Gegenwart eines wasserbindenden Mittels und/oder unter destillativer, z.B. azeotroper, Entfernung des Reaktionswassers und/oder bei erhöhter Temperatur.

Die Umsetzung mit einem reaktionsfähigen Derivat des entsprechenden Alkohols kann in üblicher Weise durchgeführt werden, ausgehend von einem Carbon-, Phosphorig-, Schweflig- oder Kohlensäureester, beispielsweise in Gegenwart eines sauren Katalysators, wie eines der vorstehend genannten, in einem inerten Lösungsmittel, wie einem aromatischen Kohlenwasserstoff, z.B. in Benzol oder Toluol, oder einem Ueberschuss des eingesetzten Alkoholderivates oder des entsprechenden Alkohols, erforderlichenfalls unter, z.B. azeotroper, Abdestillation des Reaktionswassers. Ausgehend von einem Mineralsäure- oder Sulfonsäureester setzt man die zu veresternde Säure vorteilhaft in Form eines Salzes, z.B. das Natrium-oder Kaliumsalzes und arbeitet erforderlichenfalls in Gegenwart eines basischen Kondensationsmittels, wie einer anorganischen Base, z.B. von Natrium- oder Kalium- oder Calciumhydroxid oder -carbonat, oder einer tertiären organischen Stickstoffbase, z.B. von Triethylamin oder Pyridin, erforderlichenfalls in einem inerten Lösungsmittel, wie einer der vorstehenden tertiären Stickstoffbasen, oder eines polaren Lösungsmittels, z.B. in Dimethylformamid und/oder bei erhöhter Temperatur.

Die Umsetzung mit einem Olefin kann beispielsweise in Gegenwart eines sauren Katalysators, z.B. einer Lewissäure, z.B. von Bortrifluorid, einer Sulfonsäure, z.B. von p-Toluolsulfonsäure, oder vor allem eines basischen Katalysators, z.B. von Natrium- oder Kaliumhydroxid, vorteilhaft in einem inerten Lösungsmittel, wie einem Ether, z.B. in Diethylether oder Tetrahydrofuran, erfolgen.

Eine freie Carboxylgruppe R kann ferner durch Umsetzung mit Ammoniak oder einem mindestens ein Wasserstoffatom aufweisenden Amin in üblicher Weise unter Dehydratisierung des intermediär gebildeten

Ammoniumsalzes, z.B. durch azeotrope Destillation mit Benzol oder Toluol oder trockenes Erhitzen, in eine amidierte Carboxylgruppe R überführt werden.

Die vorstehend beschriebenen Umwandlungen freier in veresterte oder amidierte Carboxylgruppen R können aber auch so durchgeführt werden, dass man eine Verbindung der Formel I, worin R Carboxyl ist, zunächst in üblicher Weise in ein reaktionsfähiges Derivat, beispielsweise mittels eines Halogenides des Phosphors oder Schwefels, z.B. mittels Phosphortrichlorid oder -bromid, Phosphorpentachlorid oder Thionylchlorid, in ein Säurehalogenid oder durch Umsetzung mit einem entsprechenden Alkohol oder Amin in einen reaktiven Ester, d.h. Ester mit elektronenanziehenden Strukturen, wie den Ester mit Phenol, Thiophenol, p-Nitrophenol oder Cyanmethylalkohol, oder ein reaktives Amid, z.B. das von Imidazol oder 3,5-Dimethylpyrazol abgeleitete Amid, überführt und das erhaltene reaktionsfähige Derivat dann in üblicher Weise, z.B. wie nachstehend für die Umesterung, Umamidierung bzw. gegenseitige Umwandlung veresterter und amidierter Carboxylgruppen R beschrieben, mit einem entsprechenden Alkohol, Ammoniak oder dem entsprechenden mindestens ein Wasserstoffatom aufweisenden Amin zu der gewünschten Gruppe R umsetzt.

Ferner kann eine veresterte Carboxylgruppe R in üblicher Weise, z.B. durch Hydrolyse in Gegenwart eines Katalysators, beispielsweise eines basischen oder sauren Mittels, wie einer starken Base, z.B. von Natrium- oder Kaliumhydroxid, oder einer Mineralsäure, z.B. von Salzsäure, Schwefelsäure oder Phosphorsäure, zur freien Carboxylgruppe R oder z.B. durch Umsetzung mit Ammoniak oder dem entsprechenden, mindestens ein Wasserstoffatom aufweisenden Amin in eine amidierte Carboxylgruppe R überführt werden.

Eine veresterte Carboxylgruppe R kann ferner in üblicher Weise, z.B. durch Umsetzung mit einem entsprechenden Metallalkoholat, z.B. dem Natrium- oder Kaliumalkoholat des entsprechenden Alkohols, oder mit diesem selbst in Gegenwart eines Katalysators, beispielsweise einer starken Base, z.B. von Natrium- oder Kaliumhydroxid, oder einer starken Säure, wie einer Mineralsäure, z.B. von Salzsäure, Schwefelsäure oder Phosphorsäure, oder einer organischen Sulfonsäure, z.B. von p-Toluolsulfonsäure, oder einer Lewissäure, z.B. von Bortrifluorid-Etherat, zu einer anderen veresterten Carboxylgruppe R umgesetzt werden.

Eine amidierte Carboxylgruppe R kann in üblicher Weise, z.B. durch Hydrolyse in Gegenwart eines Katalysators, beispielsweise einer starken Base, wie eines Alkalimetall- oder Erdalkalimetallhydroxides oder -carbonates, z.B. von Natrium- oder Kaliumhydroxid oder -carbonat, oder einer starken Säure, wie einer Mineralsäure, z.B. von Salzsäure, Schwefelsäure oder Phosphorsäure in die freie Carboxylgruppe R umgewandelt werden.

Eine Alkenyl- bzw. Alkinylgruppe kann beispielsweise durch katalytische Hydrierung zu Niederalkyl reduziert werden, wobei die Reduktion in Gegenwart eines Hydrierungskatalysators, wie Palladium, welches gegebenenfalls auf einem Trägermaterial, wie Aktivkohle, aufgezogen ist, oder Raney-Nickel, durchgeführt wird. Die Umsetzung erfolgt vorteilhaft unter Erwärmen, z.B. auf etwa 20° bei etwa 100°C, und/oder bei erhöhtem Druck.

In einer erfindungsgemäss erhältlichen Verbindung der Formel I kann man weiterhin Niederalkanoyl $R_2$, beispielsweise durch Behandeln mit einer Base, z.B. einem Alkalimetallcarbonat, wie in 50 %iger Sodalösung, gegen Wasserstoff austauschen.

Enthält mindestens einer der Substituenten $R_2$, $R_5$ und $R_6$ als Substituenten Hydroxy, so lässt sich dieses nach an sich bekannter

Weise verethern. Die Umsetzung mit einer Alkoholkomponente, z.B. mit einem Niederalkanol, wie Ethanol, in Gegenwart von Säuren, z.B. Mineralsäure, wie Schwefelsäure, oder von Dehydratisierungsmitteln, wie Dicyclohexylcarbodiimid, führt zu Niederalkoxy. Umgekehrt kann man Ether in Alkohole abspalten, indem man die Etherspaltung mittels Säuren, wie Mineralsäuren, z.B. Halogenwasserstoffsäure, wie Bromwasserstoffsäure, oder wie Lewissäuren, z.B. Halogeniden von Elementen der 3. Hauptgruppe, wie Bortribromid, mittels eines Pyridiniumhydrohalogenids, z.B. Pyridinhydrochlorid, Thiophenol oder mittels Basen, z.B. Niederalkylaminen, wie Methylamin, durchführt.

Weiterhin lässt sich Hydroxy in Niederalkanoyloxy umwandeln, beispielsweise durch Umsetzung mit einer gewünschten Niederalkancarbonsäure, wie Essigsäure oder einem reaktionsfähigen Derivat davon, beispielsweise in Gegenwart einer Säure, wie eine Protonsäure, z.B. Chlor-, Bromwasserstoff-, Schwefel-, Phosphor- oder einer Benzolsulfonsäure, in Gegenwart einer Lewissäure, z.B. von Bortrifluorid-Etherat, oder in Gegenwart eines wasserbindenden Mittels, wie Dicyclohexylcarbodiimid. Umgekehrt kann verestertes Hydroxy, z.B. durch Basen-Katalyse, zu Hydroxy solvolysiert werden.

Erhaltene freie Verbindungen der Formel I können in an sich bekannter Weise in Salze überführt werden. Hydroxy $R_2$ und $R_5$ sowie Carboxy R werden mit entsprechenden Basen, wie Alkalimetallhydroxiden, in Salze mit Basen, oder durch Behandeln mit einer wie vorstehend aufgeführten, Oxoniumsalze bildenden Säuren in Oxoniumsalze ungewandelt werden.

Erhaltene Salze können in an sich bekannter Weise in die freien Verbindungen umgewandelt werden, z.B. durch Behandeln mit einem sauren Reagens, wie einer Mineralsäure, bzw. mit einer Base, z.B. Natronlauge.

Die Verbindungen einschliesslich ihrer Salze können auch in Form ihrer Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Endstoffes mit einer mit der racemischen Säure Salze bildenden optisch aktiven Base und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können, zerlegt. Vorteilhaft isoliert man den wirksameren der beiden Antipoden.

Die obigen Reaktionen werden in üblicher Weise in An- oder Abwesenheit von Lösungs-, Verdünnungs-, Kondensations- und/oder katalytischen Mitteln, falls notwendig, bei erniedrigter oder erhöhter Temperatur, im geschlossenen Gefäss und/oder in einer Inertgasatmosphäre durchgeführt.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt und/oder einen Ausgangsstoff in Form eines Salzes, Derivates und/oder Racemates bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, ihre Verwendung, z.B. als Arzneimittelwirkstoffe, pharmazeutische Präparate, die solche Ausgangsstoffe enthalten, Formulierungsverfahren und Verfahren zu ihrer Herstellung bilden ebenfalls einen Gegenstand der vorliegenden Erfindung. Die Erfindung betrifft dabei insbesondere solche Ausgangsstoffe, z.B. der Formeln IIa, III, IV und V, die zu den bevorzugten Verbindungsgruppen der Formel I führen.

Die Erfindung betrifft ferner pharmazeutische Präparate, die Wirkstoffe der Formel I bzw. geeignete Ausgangsstoffe enthalten, sowie Verfahren zu ihrer Herstellung.

Bei den erfindungsgemässen pharmazeutischen Präparaten handelt es sich um solche zur enteralen, wie oralen, nasalen oder rektalen, sowie zur parenteralen oder buccalen Verabreichung an Warmblütern. Die Dosierung des Wirkstoffs, der alleine oder zusammen mit dem üblichen Träger- und Hilfsmaterial appliziert wird, hängt von der Warmblüterspezies, deren Alter und dem individuellen Zustand sowie der Applikationsweise ab. Die Dosis, die einem Warmblüter von etwa 70 kg Körpergewicht verabreicht wird, beträgt, z.B. bei oraler Applikation, von etwa 100 mg bis etwa 1000 mg, vorzugsweise von etwa 250 mg bis etwa 750 mg.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/ oder Calciumphosphate, z.B. Tricalciumphosphat oder Calciumhydrogenphosphat, ferner Bindemittel, wie Stärkekleister und Verwendung z.B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Ueberzügen versehen, wobei man u.a. konzentrierte Zuckerlösungen, welche gegebenenfalls arabischen Gummi, Talk, Polyvinylpyrrolidon, Polyethylenglycol und/oder Titandioxid enthalten. Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet.
Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Pharmazeutische Präparate für topische und lokale Verwendung, z.B. für die Behandlung der Haut, sind Lotionen und Cremen, die eine flüssige oder semifeste Oel-in-Wasser- oder Wasser-in-Oel-Emulsion enthalten, und Salben (wobei solche vorzugsweise ein Konservierungsmittel enthalten).

Inhalationspräparate für die Behandlung der Atemwege durch nasale oder buccale Verabreichung sind z.B. Aerosole oder Sprays, welche den pharmakologischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise ein flüssiges Treibgas mit einem Siedepunkt unter der Raumtemperatur, sowie, wenn erwünscht, Trägerstoffe, wie flüssige oder feste, nicht-ionische oder anionische oberflächenaktive Mittel und oder feste Verdünnungsmittel. Präparate, in welchen der pharmakologische Wirkstoff in Lösung vorliegt, enthalten ausser diesen ein geeignetes Treibmittel, ferner, falls notwendig, ein zusätzliches Lösungsmittel und/oder einen Stabilisator. Anstelle des Treibgases kann auch Druckluft verwendet werden.

Die erfindungsgemässen pharmazeutischen Inhalationspräparate sind beispielsweise Aerosol-, vorzugsweise Feststoffaerosolbildende Gemische, d.h. Gemische, die mittels ihres eigenen Dampfdruckes oder des Druckes eines komprimierten Gases, wie Druckluft, Lachgas oder Kohlendioxid, inhalierbare Aerosole, vorzugsweise inhalierbare Feststoffaerosole bilden können.

Die Erfindung betrifft ebenfalls die Verwendung der neuen Verbindungen der Formel I und ihrer Salze als pharmakologisch aktive Verbindungen, insbesondere als Antiallergika, vorzugsweise in Form von pharmazeutischen Präparaten.
Die nachfolgenden Beispiele illustrieren die oben beschriebene Erfindung; sie sollen jedoch diese in ihrem Umfang in keiner Weise einschränken. Temperaturen werden in Celsiusgraden angegeben.

BAD ORIGINAL

Beispiel 1: 10 g eines Gemisches aus β-Methylumbelliferonyl-7-maleinsäure und β-Methylumbelliferonyl-7-fumarsäure wird zusammen mit 100 g Polyphosphorsäure 1 Std. bei 120° gerührt und auf Eiswasser gegossen. Das dabei ausgefallene Produkt wird abgenutscht, mit Wasser, wenig Ethanol und viel Ether nachgewaschen und unter Hochvakuum bei 80° getrocknet. Man erhält so ein Gemisch aus β-Methylumbelliferonyl-7-maleinsäure, 4-Methyl-2,6-dioxo-2H,6H-benzo[1,2-b:5,4-b']dipyran-8-carbonsäure und 4-Methyl-2,10-dioxo-2H,10H-benzo[1,2-b:5,6-b']dipyran-8-carbonsäure. Aus diesem Gemisch wird die β-Methylumbelliferonyl-7-maleinsäure durch mehrmaliges Aufschlämmen in heissem Methanol und anschliessendem Abnutschen abgetrennt. Die im Nutschgut verbleibenden Benzopyrandicarbonsäuren werden an 200 g Silicagel chromatographiert. Man erhält so die 4-Methyl-2,10-dioxo-2H,10H-benzo[1,2-b:5,6-b']-dipyran-8-carbonsäure vom Smp. 275-277° und die 4-Methyl-2,6-dioxo-2H,6H-benzo[1,2-b:5,4-b']-dipyran-8-carbonsäure vom Smp. 301-303°.

Das Ausgangsmaterial kann beispielsweise folgendermassen hergestellt werden:

Das Reaktionsgemisch aus 48 g β-Methylumbelliferon, 32 ml Acetylendicarbonsäuredimethylester, 2 ml Benzyltrimethylammoniumhydroxid und 900 ml Dioxan wird etwa 50 Min. bei 100° gerührt, nochmals mit 33 ml Acetylendicarbonsäuredimethylester und einer Spatelspitze Kaliumcarbonat versetzt und nach weiteren 40 Min. Rühren auf 20° abgekühlt und direkt wie folgt verseift. Die Mischung wird mit 180 ml 25%-iger Natronlauge und 800 ml Wasser versetzt und 5 Std. bei 100° gerührt. Nach dem Abkühlen wird filtriert und das Filtrat eingedampft. Der Rückstand wird zwischen Wasser und Ethylacetat verteilt, und aus den wässrigen Phasen erhält man nach Ansäuern und Extrahieren mit Ether ein rohes Gemisch aus β-Methylumbelliferonyl-7-maleinsäure und β-Methylumbelliferonyl-7-fumarsäure, welches ohne Trennung weiterverarbeitet wird.

Beispiel 2: 5 g eines Gemisches von 4-Methyl-2,6-dioxo-2H,6H-benzo[1,2-b:5,4-b']dipyran-8-carbonsäure und 4-Methyl-2,10-dioxo-2H,10H-benzo[1,2-b:5,6-b']dipyran-8-carbonsäure werden zusammen mit 150 ml Methanol und 0,4 ml konz. Schwefelsäure 15 Std. unter Rückfluss erhitzt. Danach wird das Reaktionsgemisch auf 0° abgekühlt, abgenutscht und mit wenig Methanol und Ether nachgewaschen. Das Nutschgut wird an 200 g Silicagel mit Chloroform chromatographiert. Zuerst wird der 4-Methyl-2,6-dioxo-2H,6H-benzo[1,2-b:5,4-b']dipyran-8-carbonsäuremethylester eluiert (Smp. 308-310° nach Umkristallisieren aus Chloroform/Methanol). Aus den nachfolgenden Fraktionen erhält man den 4-Methyl-2,10-dioxo-2H,10H-benzo[1,2-b:5,6-b']-dipyran-8-carbonsäuremethylester vom Smp. 269-270° (aus Methanol/Chloroform).

Beispiel 3: 2 g 4-Methyl-2,6-dioxo-2H,6H-benzo[1,2-b:5,4-b']-dipyran-8-carbonsäuremethylester werden zusammen mit 25 ml Eisessig und 12 ml konz. Salzsäure 5 Std. unter Rückfluss erhitzt. Danach wird das Reaktionsgemisch auf Raumtemperatur abgekühlt und abgenutscht. Das Nutschgut wird mit wenig Alkohol und mit Ether gewaschen, getrocknet und anschliessend aus Chloroform umkristallisiert. Man erhält so die 4-Methyl-2,6-dioxo-2H,6H-benzo[1,2-b:5,4-b']-dipyran-8-carbonsäure vom Smp. 301-303°.

In analoger Weise kann man ferner herstellen 4-Methyl-2,10-dioxo-2H,10H-benzo[1,2-b:5,6-b']-dipyran-8-carbonsäure, Smp. 275-277°.

Beispiel 4: Eine Suspension von 12,6 g 6-(3-Ethoxycarbonyl-3-oxo-propionyl)-7-hydroxy-4-methyl-8-propyl-2-oxo-2H-benzo[1,2-b]-pyran in 85 ml Essigsäure und 17 ml konz. Salzsäure wird 6 Std. unter Rückfluss erhitzt. Anschliessend wird abgekühlt und mit 600 ml Wasser verdünnt. Die feinen Kristalle werden abgesaugt, mit Wasser gewaschen und im Hochvakuum getrocknet. Man erhält die 4-Methyl-10-propyl-2,6-dioxo-2H,6H-benzo[1,2-b:5,4-b']-dipyran-8-carbonsäure vom Smp. 294-295°.

Das Ausgangsmaterial kann beispielsweise folgendermassen hergestellt werden: Eine Lösung von 100 g 8-Allyl-7-hydroxy-4-methylcumarin in 1,5 l Tetrahydrofuran wird unter Zusatz von 10 g Raney-Nickel etwa 90 Min. bei Raumtemperatur mit Wasserstoff hydriert. Der Katalysator wird anschliessend abgenutscht und das Reaktionsgemisch eingedampft. Nach Umkristallisation aus Ethanol/Wasser erhält man das 7-Hydroxy-4-methyl-8-propyl-cumarin vom Smp. 180-182°. Zu einer Suspension von 21,8 g 7-Hydroxy-4-methyl-8-propyl-cumarin in 75,6 ml Essigsäureanhydrid wird ein Tropfen konz. Schwefelsäure gegeben und bei einer Oelbadtemperatur von 85° etwa 2 Std. gerührt. Das Reaktionsgemisch wird abgekühlt und auf etwa 500 ml Eis gegossen. Das ausgefallene Produkt wird abgenutscht und mit Wasser gewaschen. Nach Umkristallisation aus Methanol/Wasser erhält man das 7-Acetoxy-4-methyl-8-propyl-cumarin vom Smp. 98-99°.

Eine Mischung von 20 g 7-Acetoxy-4-methyl-8-propyl-cumarin und 25,6 g Aluminiumchlorid werden ohne zu Rühren mit einem Oelbad auf 120° erhitzt. Nach etwa 15 Min. wurde das Oelbad auf 170° gestellt und das Reaktionsgemisch etwa 1 Std. bei dieser Temperatur stehengelassen. Danach wird abgekühlt und mit 250 ml Eis und 1 ml konz. Salzsäure hydrolysiert. Nach 30 minütigem starkem Rühren wird das Produkt abgenutscht und mit Wasser gewaschen. Nach Umkristallisation aus Methanol/Wasser erhält man das 6-Acetyl-7-hydroxy-4-methyl-8-propyl-cumarin vom Smp. 117-118°.

Zu einer frisch hergestellten Natriumethanolatlösung aus 2,42 g Natrium und 105 ml absolutem Ethanol werden 8,12 ml Oxalsäurediethylester gegeben und etwa 1 Std. bei Raumtemperatur gerührt. Dann werden 13 g 6-Acetyl-7-hydroxy-4-methyl-8-propyl-cumarin zugegeben und noch 30 Min. unter Rückfluss erhitzt. Nach dem Abkühlen wird auf

100 ml Eisessig und 800 ml Wasser gegossen. Das Produkt wird mit Chloroform extrahiert. Die Chloroformphasen werden anschliessend mit Wasser dreimal gewaschen, vereinigt, über Natriumsulfat getrocknet und am Rotationsverdampfer unter Vakuum eingedampft. Es wird 6-(3-Ethoxycarbonyl-3-oxo-propionyl)-7-hydroxy-4-methyl-8-propyl-2-oxo-2H-benzo [1,2-b]-pyran erhalten, das ohne weitere Reinigung eingesetzt wird.

In analoger Weise erhält man nach Umkristallisieren aus Ethanol 8-(3-Ethoxycarbonyl-3-oxo-propionyl)-7-hydroxy-4-methyl-2-oxo-2H-benzo [3,4-b]-pyran vom Smp. 153-155°.

Beispiel 5: In analoger Weise wie in Beispiel 4 erhält man die 4-Methyl-6-propyl-2,10-dioxo-2H,10H-benzo[1,2-b:5,6-b']-dipyran-8-carbonsäure vom Smp. 125-127°.

Das Ausgangsmaterial kann folgendermassen hergestellt werden: Eine Suspension von 43,6 g 8-Acetyl-7-hydroxy-4-methyl-cumarin, 41,5 g Kaliumcarbonat und 18,9 ml Allylbromid in 1,6 l absolutem Aceton wird etwa 8 Std. unter Rückfluss gekocht. Nach Abkühlen wird 1,2 l Wasser zugesetzt und das Aceton am Rotationsverdampfer unter Vakuum eingedampft. Das ausgefallene Produkt wird abfiltriert und aus Methanol/Wasser umkristallisiert. Man erhält so das 8-Acetyl-4-methyl-7-allyloxy-2-oxo-2H-benzo[1,2-b]-pyran vom Smp. 119-120°.

50,7 g 8-Acetyl-4-methyl-7-allyloxy-2-oxo-2H-benzo[1,2-b]-pyran werden 3 Std. ohne zu rühren bei einer Badtemperatur von 205° gehalten. Durch anschliessende chromatographische Reinigung an Kieselgel mit dem Laufmittelsystem Toluol/Essigester (10:1) wird 8-Acetyl-7-hydroxy-4-methyl-6-allyl-2-oxo-2H-benzo[1,2-b]-benzopyran erhalten mit dem Smp. 131-133°.

In analoger Weise wie in Beispiel 4 erhält man durch katalytische Hydrierung das 8-Acetyl-7-hydroxy-4-methyl-6-propyl-2-oxo-2H-benzo[1,2-b]-pyran vom Smp. 129-131°.

In analoger Weise wie in Beispiel 4 erhält man aus 8-Acetyl-7-hydroxy-4-methyl-6-propyl-2-oxo-2H-benzo[1,2-b]-pyran das 8-(3-Ethoxycarbonyl-3-oxo-propionyl)-7-hydroxy-4-methyl-6-propyl-2-oxo-2H-benzo [1,2-b]-pyran, welches als Rohprodukt weiterverwendet wird.

Beispiel 6: In analoger Weise wie in Beispiel 4 erhält man ausgehend von 6-(3-Ethoxycarbonyl-3-oxo-propionyl)-7-hydroxy-4-methyl-8-allyl-2-oxo-2H-benzo[1,2-b]-pyran die 4-Methyl-10-allyl-2,6-dioxo-2H,6H-benzo[1,2-b:5,4-b']-dipyran-8-carbonsäure, Smp. 295-297°.

Das Ausgangsmaterial kann beispielsweise folgendermassen hergestellt werden: Eine Mischung von 109 g 7-Acetoxy-4-methyl-2-oxo-2H-benzo[1,2-b]pyran [A.Russel, J.R. Frye, W.L. Mauldin, J. Amer. Chem. Soc. 62, 1441 (1940)] und 167 g Aluminiumchlorid wurden ohne zu Rühren mit einem 120° warmen Oelbad geheizt. Nach ca. 15 Minuten wurde das Oelbad auf 170° gestellt und 1 Std. bei dieser Temperatur stehen gelassen. Danach wurde abgekühlt und mit 2 l Eis und 10 ml konz. HCl hydrolisiert. Nach ca. 30 minütigem starken Rühren wurde das Produkt abgenutscht und mit Wasser gewaschen. Umkristallisation aus Methanol/Wasser ergab ein Gemisch der beiden Isomeren o-Hydroxyacetophenone. Durch Chromatographie am Kieselgel mit dem Fliessmittelsystem Toluol/Essigester = 6:1 konnten die beiden isomeren Produkte voneinander getrennt werden. So wurden 8-Acetyl-7-hydroxy-4-methylcumarin, Smp. 163 - 164° und 6-Acetyl-7-hydroxy-4-methylcumarin, Smp. 206-207° erhalten. In analoger Weise wie in Beispiel 5 erhält man ausgehend von 6-Acetyl-7-hydroxy-4-methyl-2-oxo-2H-benzo[1,2-b]-pyran und Allylbromid das 6-Acetyl-4-methyl-7-allyloxy-2-oxo-2H-benzo[1,2-b]-pyran, Smp. 155-6°.

In analoger Weise wie in Beispiel 5 erhält man ausgehend von 6-Acetyl-4-methyl-7-allyloxy-2-oxo-2H-benzo[1,2-b]-pyran das 6-Acetyl-7-hydroxy-4-methyl-8-allyl-2-oxo-2H-benzo[1,2-b]-pyran, Smp. 116-118°.

In analoger Weise wie in Beispiel 4 erhält man ausgehend von 6-Acetyl-7-hydroxy-4-methyl-8-allyl-2-oxo-2H-benzo[1,2-b]-pyran das 6-(3-Ethoxycarbonyl-3-oxo-propionyl)-7-hydroxy-4-methyl-8-allyl-2-oxo-2H-benzo[1,2-b]-pyran.

Analog hierzu erhält man ausgehend von 8-Acetyl-7-hydroxy-4-methyl-6-propen-2-yl-2-oxo-2H-benzo[1,2-b]-pyran das 8-(3-Ethoxycarbonyl-3-oxo-propionyl)-7-hydroxy-4-methyl-6-propen-2-yl-2-oxo-2H-benzo[5,4-b]-pyran.

Beispiel 7: In analoger Weise wie in Beispiel 5 beschrieben kann man ferner herstellen:
4-Methyl-2,10-dioxo-2H,10H-benzo[1,2-b:5,6-b']-dipyran-8-carbonsäure (monohydrat) vom Smp. 275-77°,
6-Allyl-4-methyl-2,10-dioxo-2H,10H-benzo[1,2-b:5,6-b']-dipyran-8-carbonsäure Smp. 143-5°.

Beispiel 8: Eine Lösung von 3,6 g 6-(3-Ethoxycarbonyl-3-oxo-propionyl)-7-hydroxy-4-methyl-8-propyl-2-oxo-2H-benzo[1,2-b]-pyran in 150 ml abs. Ethanol und 25 ml konz. Salzsäure wird 30 Min. unter Rückfluss erhitzt. Das Reaktionsgemisch wird abgekühlt und das Produkt abfiltriert und mit Ethanol und Ether gewaschen. Man erhält so den 4-Methyl-10-propyl-2,6-dioxo-2H,6H-benzo[1,2-b:5,4-b']-dipyran-8-carbonsäureethylester vom Smp. 173-175°.

In analoger Weise kann man ferner herstellen: 4-Methyl-2,10-dioxo-2H,10H-benzo[1,2-b:5,6-b']-dipyran-8-carbonsäureethylester vom Smp. 271-273°,
4-Methyl-6-allyl-2,10-dioxo-2H,10H-benzo[1,2-b:5,6-b']-dipyran-8-carbonsäure-ethylester vom Smp. 145-147°,
4-Methyl-6-propyl-2,10-dioxo-2H,10H-benzo[1,2-b:5,6-b']-dipyran-8-carbonsäure-ethylester vom Smp. 81-83°,
4-Methyl-10-allyl -2,6-dioxo-2H,6H-benzo[1,2-b:5,4-b']-dipyran-8-carbonsäure-ethylester, Smp. 152-154°.

Beispiel 9: Eine Lösung aus 21,8 g 7-Hydroxy-4-methyl-8-propyl-cumarin, 14,7 ml Acetylendicarbonsäuredimethylester und 2,3 ml 40%-iges Trimethylammoniumhydroxid (Triton B) in 250 ml abs. Dioxan wird 90 Min. unter Rückfluss gekocht. Das Reaktionsgemisch wird anschliessend am Rotationsverdampfer unter Vakuum eingeengt und der Rückstand zwischen Essigester und Wasser verteilt. Die Wasserphase wird abgetrennt und nochmals mit Essigester extrahiert. Die vereinigten organischen Phasen werden mit Wasser zweimal gewaschen, über Natriumsulfat getrocknet und am Rotationsverdampfer unter Vakuum eingedampft. Der Rückstand wird mit 500 ml Essigsäure, 200 ml konz. Salzsäure und 300 ml Wasser versetzt und ca. 20 Min. unter Rückfluss erhitzt. Anschliessend wird abgekühlt, mit 1500 ml Wasser verdünnt und zweimal mit Essigester extrahiert. Die Essigester-Extrakte werden zweimal mit Wasser gewaschen, vereinigt, über Natriumsulfat getrocknet und am Rotationsverdampfer unter Vakuum eingedampft. Das rohe cis/trans-Dicarbonsäure-Rohprodukt wird eingeengt, mit 250 ml Chlorsulfonsäure versetzt und etwa 1 Std. bei Raumtemperatur gerührt. Das schwarze Reaktionsgemisch wird vorsichtig auf ca. 3 l Eis gegossen. Der braune Niederschlag wird abgenutscht, mit Wasser gewaschen und im Hochvakuum getrocknet. Der Rückstand wird anschliessend in Aceton gelöst und bis auf etwa 50 ml am Rotationsverdampfer unter Vakuum eingedampft, dann mit 300 ml Essigester versetzt und der fein-kristalline Niederschlag abgenutscht. Man erhält so die 4-Methyl-10-propyl-2,6-dioxo-2H,6H-benzo[1,2-b:5,4-b']-dipyran-8-carbonsäure vom Smp. 295-297°

Beispiel 10: Eine auf ca. 0° gekühlte Lösung von 3,12 g (10,0 mMol) 4-Methyl-10-allyl-2,6-dioxo-2H,6H-benzo[1,2-b:5,4-b']-dipyran-8-carbonsäure (Beispiel 6) in 25 ml absolutem Dimethylformamid wird mit 1,75 ml (11,0 mMol) Iodmethylpivalat und 1,64 g (11,0 mMol) 1,5-Diazabicyclo[5.4.0]undec-5-en versetzt. Das Kühlbad wird entfernt und noch 1 Stunde bei Raumtemperatur gerührt. Das Reaktionsgemisch wird mit Essigester verdünnt, mit Wasser gewaschen und über Natriumsulfat getrocknet. Das Lösungsmittel wird am Vakuumrotationsverdampfer

eingeengt und der Rückstand aus Methanol/Wasser umkristallisiert. Es resultiert der 10-Allyl-4-methyl-2,6-dioxo-2H,6H-benzo-[1,2-b:5,4-b']-dipyran-8-carbonsäurepivaloyloxymethylester vom Smp. 99 - 101°.

Beispiel 11: In analoger Weise wie in Beispiel 8 beschrieben kann man 2,10-Dioxo-4-methyl-6-[3-(1-butenyl)]-2H,10H-benzo[1,2-b:5,6-b']-dipyran-8-carbonsäureethylester herstellen, Smp. 185-187°, ausgehend von 6-[3-(1-Butenyl)]-8-(3-ethoxycarbonyl-3-oxo-propionyl)-7-hydroxy-4-methyl-2-oxo-2H-benzo[3,4-b]-pyran.

Das Ausgangsmaterial kann z.B. folgendermassen hergestellt werden:

8-Acetyl-7-[2-butenyloxy]-4-methyl-2-oxo-2H-benzo[1,2-b]-pyran analog Beispiel 5, Smp. 131 - 133°, ausgehend von 8-Acetyl-7-hydroxy-4-methyl-2-oxo-2H-benzo[1,2-b]-pyran und 4-Brom-but-2-en.

8-Acetyl-6-[3-(1-butenyl)]-7-hydroxy-4-methyl-2-oxo-2H-benzo[1,2-b]-pyran, Smp. 116 - 118°, ausgehend von 8-Acetyl-7-[2-butenyloxy]-4-methyl-2-oxo-2H-benzo[1,2-b]-pyran, in Analogie zu Beispiel 5.

6-[3-(1-Butenyl)]-8-(3-ethoxycarbonyl-3-oxo-propionyl)-7-hydroxy-4-methyl-2-oxo-2H-benzo[3,4-b]-pyran, ausgehend von 8-Acetyl-6-[3-(1-butenyl)]-7-hydroxy-4-methyl-2-oxo-2H-benzo[1,2-b]-pyran, in Analogie zu Beispiel 4.

Beispiel 12: In analoger Weise wie in Beispiel 4 beschrieben kann man 2,10-Dioxo-4-methyl-6-[3-(1-butenyl)]-2H,10H-benzo[1,2-b:5,6-b']-dipyran-8-carbonsäure, Smp. 248 - 250°, erhalten, ausgehend von 6-[3-(1-Butenyl)]-8-(3-ethoxycarbonyl-3-oxo-propionyl)-7-hydroxy-4-methyl-2-oxo-2H-benzo[3,4-b]pyran.

Beispiel 13: In analoger Weise wie in Beispiel 8 beschrieben kann man 2,10-Dioxo-4-methyl-6-[2-(n-butyl)]-2H-10H-benzo[1,2-b:5,6-b']-dipyran-8-carbonsäureethylester herstellen, Smp. 177 - 178°, ausgehend von 6-[2-(n-Butyl)]-8-(3-ethoxycarbonyl-3-oxo-propionyl)-7-hydroxy-4-methyl-2-oxo-2H-benzo[3,4-b]-pyran.

Das Ausgangsmaterial kann z.B. folgendermassen hergestellt werden:

8-Acetyl-6-[2-(n-butyl)]-7-hydroxy-4-methyl-2-oxo-2H-benzo[1,2-b]-pyran, Smp. 97 - 99°, analog Beispiele 4 und 5, ausgehend von 8-Acetyl-6-[3-(1-butenyl)]-7-hydroxy-4-methyl-2-oxo-2H-benzo[1,2-b]-pyran (s. Beispiel 11).

6-[2-(n-Butyl)]-8-(3-ethoxycarbonyl-3-oxo-propionyl)-7-hydroxy-4-methyl-2-oxo-2H-benzo[3,4-b]-pyran, ausgehend von 8-Acetyl-6-[2-(n-butyl)]-7-hydroxy-4-methyl-2-oxo-2H-benzo[1,2-b]-pyran in Analogie zu Beispiel 4.

Beispiel 14: In analoger Weise wie in Beispiel 4 beschrieben kann man 2,10-Dioxo-4-methyl-6-[2-(n-butyl)]-2H,10H-benzo[1,2-b:5,6-b']-dipyran-8-carbonsäure herstellen, Smp. 237 - 239°, ausgehend von 6-[2-(n-Butyl)]-8-(3-ethoxycarbonyl-3-oxo-propionyl)-7-hydroxy-4-methyl-2-oxo-2H-benzo[3,4-b]-pyran.

Beispiel 15: Tabletten, enthaltend 25 mg Wirkstoff, z.B. 4-Methyl 10-propyl-2,6-dioxo-2H,6H-benzo[1,2-b:5,4-b']dipyran-8-carbonsäure oder ein Salz davon, können folgendermassen hergestellt werden:

Bestandteile (für 1000 Tabletten):

| | |
|---|---|
| Wirkstoff | 25,0 g |
| Lactose | 100,7 g |

| | |
|---|---|
| Weizstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung: Sämtliche feste Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

In analoger Weise können auch Tabletten enthaltend eine der in den Beispielen 1 - 14 genannten Verbindungen der Formel I hergestellt werden.

Beispiel 16: Eine zur Inhalation geeignete, etwa 2%ige wässrige Lösung eines in freier Form oder in Formel eines Salzes wasserlöslichen erfindungsgemässen Wirkstoffs kann z.B. in folgender Zusammensetzung hergestellt werden:

Zusammensetzung

| | | |
|---|---|---|
| Wirkstoff, z.B. 4-Methyl-10-allyl-2,6-dioxo-2H,6H-benzo[1,2-6:5,4-b']dipyran-8-carbonsäureethylester | | 2000 mg |
| Stabilisator, z.B. Ethylendiamintetraessigsäure-Dinatriumsalz | | 10 mg |
| Konservierungsmittel, z.B. Benzalkoniumchlorid, | | 10 mg |
| Wasser, frisch destilliert, | ad | 100 ml |

Herstellung

Der Wirkstoff wird unter Zusatz der äquimolekularen Menge 2n-Natronlauge in frisch destilliertem Wasser gelöst. Dann wird der Stabilisator und das Konservierungsmittel hinzugegeben. Nach vollständiger Auflösung aller Komponenten wird die erhaltene Lösung auf 100 ml aufgefüllt, in Fläschchen abgefüllt und diese gasdicht verschlossen.

In analoger Weise können auch 2%-ige Inhalationslösungen enthaltend einen Wirkstoff eines der Beispiele 1 - 14 hergestellt werden.

Beispiel 17: Eine zur Inhalation geeignete, etwa 2 %-ige wässrige Lösung eines in freier Form oder in Form des Natriumsalzes wasserlöslichen erfindungsgemässen Wirkstoff kann z.B. in folgender Zusammensetzung hergestellt werden:

Zusammensetzung

| | | |
|---|---|---|
| Wirkstoff, z.B. 4-Methyl-10-allyl-2,6-dioxo-2H,6H-benzo[1,2-b:5,4-b']dipyran-8-carbonsäurethylester | | 2000 mg |
| Stabilisator, z.B. Aethylendiamintetraessigsäure-Dinatriumsalz | | 10 mg |
| Konservierungsmittel, z.B. Benzalkoniumchlorid, | | 10 mg |
| Wasser, frisch destilliert, | ad | 100 ml |

Herstellung

Der Wirkstoff wird in frisch destilliertem Wasser gelöst. Dann wird der Stabilisator und das Konservierungsmittel hinzugegeben. Nach vollständiger Auflösung aller Komponenten wird die erhaltene Lösung auf 100 ml aufgefüllt, in Fläschchen abgefüllt und diese gasdicht verschlossen.

In analoger Weise können auch 2%-ige Inhalationslösungen enthaltend einen anderen Wirkstoff eines der Beispiele 1 bis 14 hergestellt werden.

Beispiel 18: Zur Insufflation geeignete, etwa 25 mg eines erfindungsgemässen Wirkstoffes enthaltende Kapseln können z.B. in folgender Zusammensetzung hergestellt werden:

Zusammensetzung

| | |
|---|---|
| Wirkstoff, z.B. 4-Methyl-10-allyl-2,6-dioxo-2H,6H-benzo[1,2-b:5,4-b']dipyran-8-carbonsäureethylester | 25 g |
| Lactose, feinst gemahlen | 25 g |

Herstellung

Der Wirkstoff und die Lactose werden innig vermischt. Das erhaltene Pulver wird sodann gesiebt und in Portionen zu je 50 mg in 1000 Gelatinekapseln abgefüllt.

In alaloger Weise können auch Insufflationskapseln enthaltend jeweils einen Wirkstoff gemäss einem der Beispiele 1 bis 14 hergestellt werden.

Patentansprüche für die Vertragsstaaten: BE, CH, DE, FR, GB, IT, NL, SE,

1. 6-substituierte 4-Oxo-4H-1-benzopyrane der Formel

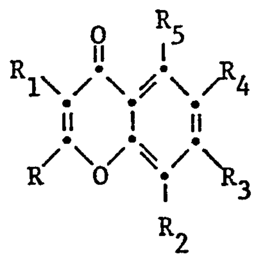

(I) ,

worin R freies, verestertes oder amidiertes Carboxy oder Tetrazolyl bedeutet, $R_1$ Wasserstoff oder einen aliphatischen Rest darstellt, $R_2$ Wasserstoff, einen aliphatischen Rest, Acyl oder gegebenenfalls verethertes oder verestertes Hydroxy bedeutet, $R_4$ eine Gruppe der Formel $-C(R_6)=C(R_7)-C(=O)-R_8$ darstellt, in der $R_6$ Wasserstoff oder einen gegebenenfalls durch Hydroxy substituierten aliphatischen Rest und $R_7$ Wasserstoff oder einen aliphatischen Rest bedeutet und $R_8$ gemeinsam mit einem der Reste $R_3$ und $R_5$ Oxy darstellen und der andere der Reste $R_3$ und $R_5$ Wasserstoff oder, im Falle von $R_5$, auch Hydroxy bedeutet, und Salze von Verbindungen der Formel I mit salzbildenden Eigenschaften.

2. Verbindungen gemäss Anspruch 1 der Formel I mit der Massgabe, dass R von Ethoxycarbonyl verschieden ist, wenn $R_4$ eine Gruppe der Formel $-C(R_6)=C(R_7)-C(=O)-R_8$ bedeutet, $R_8$ gemeinsam mit $R_3$ Oxy bedeutet, $R_1$, $R_2$, $R_5$ und $R_7$ Wasserstoff darstellen und $R_6$ Methyl ist.

3. Verbindungen gemäss Anspruch 1 oder 2 der Formel I, worin R Carboxy, Niederalkoxycarbonyl, Hydroxyniederalkoxycarbonyl, Niederalkanoyloxyniederalkoxycarbonyl, Carbamoyl, N-Mono- oder N-Diniederalkylcarbamoyl, 1-Tetrazolyl-carbonyl oder 5-Tetrazolyl bedeutet, $R_1$ Wasserstoff, Niederalkyl oder Niederalkenyl ist, $R_2$ Wasserstoff, Niederalkyl, Niederalkenyl, Niederalkanoyl, gegebenenfalls durch Niederalkyl, Niederalkoxy und/oder Halogen substituiertes Benzoyl, Hydroxy, Niederalkoxy, Niederalkenyloxy, Halogen oder Niederalkanoyloxy darstellt, $R_4$ eine Gruppe der Formel $-C(R_6)=C(R_7)-C(=O)-R_8$ bedeutet, in der $R_6$ Wasserstoff, Niederalkyl, Niederalkenyl oder Hydroxyniederalkyl und $R_7$ Wasserstoff, Niederalkyl oder Niederalkenyl bedeutet, und $R_8$ gemeinsam mit einem der Reste $R_3$ und $R_5$ Oxy bedeutet und der andere der Reste $R_3$ und $R_5$ Wasserstoff oder, im Falle von $R_5$, auch Hydroxy bedeutet, und deren Salze.

4. Verbindungen gemäss Anspruch 1 oder 2 der Formel I, worin R Carboxy, Niederalkoxycarbonyl oder Niederalkanoyloxyniederalkoxycarbonyl bedeutet, $R_1$ Wasserstoff oder Niederalkyl darstellt, $R_2$ Wasserstoff, Niederalkyl, Niederalkenyl, Hydroxy, Niederalkoxy, Niederalkanoyloxy oder Halogen bedeutet, $R_4$ eine Gruppe der Formel $-C(R_6)=C(R_7)-C(=O)-R_8$ darstellt, in der $R_6$ und $R_7$ unabhängig voneinander Wasserstoff oder Niederalkyl bedeuten und $R_8$ gemeinsam mit einem der Reste $R_3$ und $R_5$ Oxy bedeutet und der andere der Reste $R_3$ und $R_5$ Wasserstoff ist, und deren Salze.

5. Verbindungen gemäss Anspruch 1 oder 2 der Formel I, worin R Carboxy oder Niederalkoxycarbonyl mit bis und mit 4 C-Atomen im Nieder-

alkylteil bedeutet, $R_1$ für Wasserstoff steht, $R_2$ Wasserstoff, Niederalkyl mit bis und mit 4 C-Atomen oder Niederalkenyl mit bis und mit 4 C-Atomen bedeutet, $R_4$ eine Gruppe der Formel $-C(R_6)=C(R_7)-C(=O)-R_8$ darstellt, in der $R_6$ Niederalkyl mit bis und mit 4 C-Atomen darstellt, $R_7$ Wasserstoff bedeutet und einer der Reste $R_3$ und $R_5$ gemeinsam mit $R_8$ Oxy darstellt und der andere Wasserstoff bedeutet, und deren Salze.

6. Verbindungen gemäss Anspruch 1 oder 2 der Formel I, worin R Carboxy bedeutet, $R_1$ für Wasserstoff steht, $R_2$ Niederalkyl mit bis und mit 4 C-Atomen oder Niederalkenyl mit bis und mit 4 C-Atomen bedeutet, $R_4$ eine Gruppe der Formel $-C(R_6)=C(R_7)-C(=O)-R_8$ darstellt, in der $R_6$ Niederalkyl mit bis und mit 4 C-Atomen bedeutet, $R_7$ Wasserstoff bedeutet und einer der Reste $R_3$ und $R_5$ gemeinsam mit $R_8$ Oxy darstellt und der andere Wasserstoff bedeutet, und deren Salze.

7. 4-Methyl-2,6-dioxo-2H,6H-benzo[1,2-b:5,4-b']-dipyran-8-carbonsäure oder ein Salz davon.

8. 4-Methyl-10-propyl-2,6-dioxo-2H,6H-benzo[1,2-b:5,4-b']-dipyran-8-carbonsäure oder ein Salz davon.

9. 4-Methyl-6-propyl-2,10-dioxo-2H,10H-benzo[1,2-b:3,4-b']-dipyran-8-carbonsäure oder ein Salz davon.

10. 4-Methyl-10-allyl-2,6-dioxo-2H,6H-benzo[1,2-b:5,4-b']-dipyran-8-carbonsäure oder ein Salz davon.

11. 6-Allyl-4-methyl-2,10-dioxo-2H,10H-benzo[1,2-b:3,4-b']-dipyran-8-carbonsäure oder ein Salz davon.

12. Verbindung gemäss einem der Ansprüche 1 - 11 mit antiallergischer Wirkung.

13. Verbindung gemäss einem der Ansprüche 1 - 12 zur Anwendung in einem Verfahren zur therapeutischen Behandlung des menschlichen oder tierischen Körpers.

14. Pharmazeutische Präparate, enthaltend mindestens eine Verbindung gemäss einem der Ansprüche 1 - 13.

15. Verwendung von Verbindungen gemäss einem der Ansprüche 1 - 13 zur Behandlung allergischer Erkrankungen.

16. Verwendung von Verbindungen gemäss einem der Ansprüche 1 - 13 zur Herstellung pharmazeutischer Präparate.

17. Die in den Beispielen 1 - 14 genannten neuen Verbindungen.

18. Verfahren zur Herstellung pharmazeutischer Präparate, dadurch gekennzeichnet, dass man eine Verbindung gemäss einem der Ansprüche 1 - 13, gegebenenfalls unter Beimischung von üblichen Hilfs- und Trägerstoffen, zu pharmazeutischen Präparaten verarbeitet.

19. Verfahren zur Herstellung von Verbindungen der Formel I gemäss einem der Ansprüche 1 - 13, dadurch gekennzeichnet, dass man

a) Verbindungen der Formel

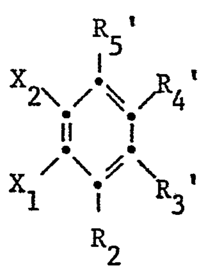

(IIa) ,

worin $X_1$ Hydroxy oder reaktionsfähiges verestertes Hydroxy bedeutet, $X_2$ eine Gruppe der Formel $-CO-CH(R_1)-CO-R$ bedeutet oder worin $X_1$ eine Gruppe der Formel $-O-C(R)=C(R_1)-CO-R_9$ und $R_9$ eine geeignete Abgangsgruppe bedeutet und $X_2$ Wasserstoff ist und $R_3'$, $R_4'$ bzw. $R_5'$ jeweils die Bedeutungen von $R_3$, $R_4$ bzw. $R_5$ haben, oder worin jeweils $X_1$ eine Gruppe der Formel $-O-C(R)=C(R_1)-CO-X_1'$ bedeutet und $X_1'$ mit $X_2$ eine gemeinsame Bindung bedeuten und einer der Reste $R_3'$ und $R_5'$ eine Gruppe der Formel $-O-CO-CH(R_7)-CO-R_6$ und der andere die Bedeutung von $R_3$ bzw. $R_5$ hat und $R_4'$ Wasserstoff ist, oder einer der Reste $R_3'$ und $R_5'$ Hydroxy oder reaktionsfähiges verestertes Hydroxy bedeutet und der andere die Bedeutung von $R_3$ bzw. $R_5$ hat und $R_4'$ eine Gruppe der Formel $-C(R_6)=C(R_7)-CO-R_9$ und $R_9$ eine geeignete Abgangsgruppe bedeutet, oder worin einer der Reste $R_3'$ und $R_5'$ eine Gruppe der Formel $-O-CO-C(R_7)=P(X_3)_3$ und $X_3$ einen aliphatischen oder aromatischen Rest oder Niederalkoxy bzw. gegebenenfalls substituiertes Phenoxy bedeutet und der andere die Bedeutung von $R_3$ bzw. $R_5$ hat und $R_4'$ eine Gruppe der Formel $-CO-R_6$ bedeutet, oder Salze von solchen Verbindungen cyclisiert, oder

b) aus einer Verbindung der Formel

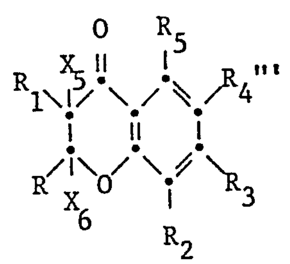

(III) ,

worin $R_4'''$ eine Gruppe der Formel $-C(R_6)(X_5')-C(R_7)(X_6')-CO-R_8$ bedeutet, die Variablen $X_5$, $X_6$, $X_5'$ und $X_6'$ jeweils Wasserstoff oder Halogen bedeuten oder eines der Variablenpaare $X_5$ und $X_6$ bzw.

$X_5'$ und $X_6'$ für eine gemeinsame Bindung steht und die Variablen des anderen Variablenpaares jeweils Wasserstoff oder Halogen bedeuten bzw. eine der Variablen des anderen Variablenpaares Wasserstoff ist und die andere eine geeignete abspaltbare Gruppe bedeutet, oder ein Salz davon unter Bildung einer oder zwei zusätzlicher Bindungen eine Verbindung der Formel $X_5' - X_6'$ und/oder $X_5 - X_6$ abspaltet, oder

c) in einer Verbindung der Formel

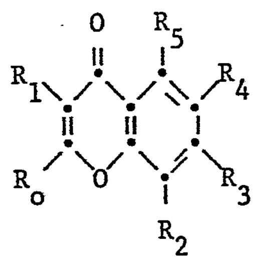

(IV),

worin $R_o$ einen in R überführbaren Rest bedeutet, $R_o$ in den Rest R überführt, oder

d) in einer Verbindung der Formel

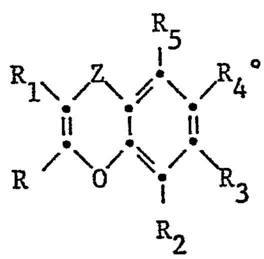

(V)

oder einem Salz oder Tautomeren davon, worin $R_4°$ für eine Gruppe der Formel $-C(R_6)=C(R_7)-Z'-R_8$ steht und eine der Gruppen Z' und Z für eine in die Carbonylgruppe überführbare Gruppe steht und die andere die Carbonylgruppe oder eine in die Carbonylgruppe überführbare Gruppe bedeutet, Z' und/oder Z in die Carbonylgruppe überführt,

erforderlichenfalls ein verfahrensgemäss erhältliches Isomerengemisch in die Isomeren auftrennt und das gewünschte Isomere isoliert und gewünschtenfalls eine so erhältliche Verbindung in eine andere Verbindung der Formel I umwandelt und/oder eine so erhältliche freie Verbindung in ein Salz oder ein so erhältliches Salz in die freie Verbindung oder ein anderes Salz überführt.

20. Verfahren nach Anspruch 19, dadurch gekennzeichnet, dass man von einer auf irgendeiner Stufe des Verfahrens als Zwischenprodukt erhältlichen Verbindung ausgeht und die fehlenden Schritte durchführt und/oder einen Ausgangsstoff in Form eines Salzes, Derivates und/oder Racemates bzw. Antipoden verwendet oder insbesondere unter den Reaktionsbedingungen bildet.

21. Die in dem Verfahren gemäss einem der Ansprüche 19 und 20 verwendeten neuen Ausgangsstoffe und/oder Zwischenprodukte.

22. Die nach dem Verfahren gemäss einem der Ansprüche 19 und 20 erhältlichen Verbindungen.

FO 7.4/DH/rz*

Patentansprüche für den Vertragsstaat AT

1. Verfahren zur Herstellung neuer 6-substituierte 4-Oxo-4H-1-benzopyrane der Formel

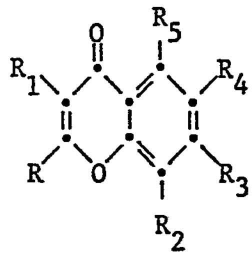

(I) ,

worin R freies, verestertes oder amidiertes Carboxy oder Tetrazolyl bedeutet, $R_1$ Wasserstoff oder einen aliphatischen Rest darstellt, $R_2$ Wasserstoff, einen aliphatischen Rest, Acyl oder gegebenenfalls verethertes oder verestertes Hydroxy bedeutet, $R_4$ eine Gruppe der Formel $-C(R_6)=C(R_7)-C(=O)-R_8$ darstellt, in der $R_6$ Wasserstoff oder einen gegebenenfalls durch Hydroxy substituierten aliphatischen Rest und $R_7$ Wasserstoff oder einen aliphatischen Rest bedeutet und $R_8$ gemeinsam mit einem der Reste $R_3$ und $R_5$ Oxy darstellen und der andere der Reste $R_3$ und $R_5$ Wasserstoff oder, im Falle von $R_5$, auch Hydroxy bedeutet, und Salze von Verbindungen der Formel I mit salzbildenden Eigenschaften, dadurch gekennzeichnet, dass man

a) Verbindungen der Formel

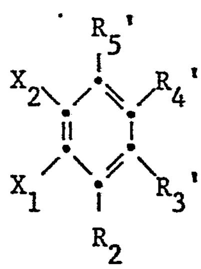

(IIa) ,

worin $X_1$ Hydroxy oder reaktionsfähiges verestertes Hydroxy bedeutet, $X_2$ eine Gruppe der Formel $-CO-CH(R_1)-CO-R$ bedeutet oder worin $X_1$ eine Gruppe der Formel $-O-C(R)=C(R_1)-CO-R_9$ und $R_9$ eine geeignete Abgangsgruppe bedeutet und $X_2$ Wasserstoff ist und $R_3'$, $R_4'$ bzw. $R_5'$ jeweils die Bedeutungen von $R_3$, $R_4$ bzw. $R_5$ haben, oder worin jeweils $X_1$ eine Gruppe der Formel $-O-C(R)=C(R_1)-CO-X_1'$ bedeutet und $X_1'$ mit $X_2$ eine gemeinsame Bindung bedeuten und einer der Reste $R_3'$ und $R_5'$ eine Gruppe der Formel $-O-CO-CH(R_7)-CO-R_6$ und der andere die Bedeutung von $R_3$ bzw. $R_5$ hat und $R_4'$ Wasserstoff ist, oder einer der Reste $R_3'$ und $R_5'$ Hydroxy oder reaktionsfähiges verestertes Hydroxy bedeutet und der andere die Bedeutung von $R_3$ bzw. $R_5$ hat und $R_4'$ eine Gruppe der Formel $-C(R_6)=C(R_7)-CO-R_9$ und $R_9$ eine geeignete Abgangsgruppe bedeutet, oder worin einer der Reste $R_3'$ und $R_5'$ eine Gruppe der Formel $-O-CO-C(R_7)=P(X_3)_3$ und $X_3$ einen aliphatischen oder aromatischen Rest oder Niederalkoxy bzw. gegebenenfalls substituiertes Phenoxy bedeutet und der andere die Bedeutung von $R_3$ bzw. $R_5$ hat und $R_4'$ eine Gruppe der Formel $-CO-R_6$ bedeutet, oder Salze von solchen Verbindungen cyclisiert, oder

b) aus einer Verbindung der Formel

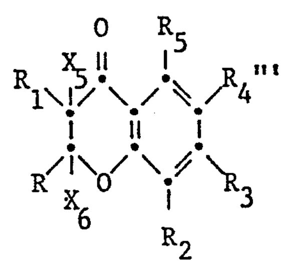

(III) ,

worin $R_4'''$ eine Gruppe der Formel $-C(R_6)(X_5')-C(R_7)(X_6')-CO-R_8$ bedeutet, die Variablen $X_5$, $X_6$, $X_5'$ und $X_6'$ jeweils Wasserstoff oder Halogen bedeuten oder eines der Variablenpaare $X_5$ und $X_6$ bzw.

$X_5'$ und $X_6'$ für eine gemeinsame Bindung steht und die Variablen des anderen Variablenpaares jeweils Wasserstoff oder Halogen bedeuten bzw. eine der Variablen des anderen Variablenpaares Wasserstoff ist und die andere eine geeignete abspaltbare Gruppe bedeutet, oder ein Salz davon unter Bildung einer oder zwei zusätzlicher Bindungen eine Verbindung der Formel $X_5' - X_6'$ und/oder $X_5 - X_6$ abspaltet, oder

c) in einer Verbindung der Formel

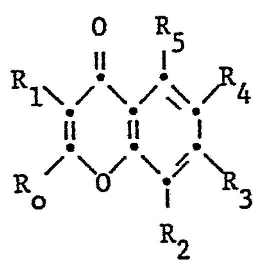

(IV),

worin $R_o$ einen in R überführbaren Rest bedeutet, $R_o$ in den Rest R überführt, oder

d) in einer Verbindung der Formel

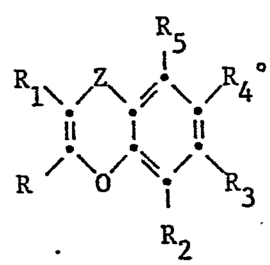

(V)

oder einem Salz oder Tautomeren davon, worin $R_4°$ für eine Gruppe der Formel $-C(R_6)=C(R_7)-Z'-R_8$ steht und eine der Gruppen Z' und Z für eine in die Carbonylgruppe überführbare Gruppe steht und die andere die Carbonylgruppe oder eine in die Carbonylgruppe überführbare Gruppe bedeutet, Z' und/oder Z in die Carbonylgruppe überführt,

erforderlichenfalls ein verfahrensgemäss erhältliches Isomerengemisch in die Isomeren auftrennt und das gewünschte Isomere isoliert und gewünschtenfalls eine so erhältliche Verbindung in eine andere Verbindung der Formel I umwandelt und/oder eine so erhältliche freie Verbindung in ein Salz oder ein so erhältliches Salz in die freie Verbindung oder ein anderes Salz überführt.

2. Verfahren nach Anspruch 1, dadurch gekennzeichnet, dass man von einer auf irgendeiner Stufe des Verfahrens als Zwischenprodukt erhältlichen Verbindung ausgeht und die fehlenden Schritte durchführt undüoder einen Ausgangsstoff in Form eines Salzes, Derivates und/oder Racemates bzw. Antipoden verwendet oder insbesondere unter den Reaktionsbedingungen bildet.

3. Verfahren nach einem der Ansprüche 1 und 2 zur Herstellung von Verbindungen der Formel I, mit der Massgabe, dass R von Ethoxycarbonyl verschieden ist, wenn $R_4$ eine Gruppe der Formel $-C(C_6)=C(R_7)-C(=O)-R_8$ bedeutet, $R_8$ gemeinsam mit $R_3$ Oxy bedeutet, $R_1$, $R_2$, $R_5$ und $R_7$ Wasserstoff darstellen und $R_6$ Methyl ist.

4. Verfahren nach einem der Ansprüche 1 bis 3, dadurch gekennzeichnet, dass man Verbindungen der Formel I, worin R Carboxy, Niederalkoxycarbonyl, Hydroxyniederalkoxycarbonyl, Niederalkanoyloxyniederalkoxycarbonyl, Carbamoyl, N-Mono- oder N-Diniederalkylcarbamoyl, 1-Tetrazolyl-carbonyl oder 5-Tetrazolyl bedeutet, $R_1$ Wasserstoff, Niederalkyl oder Niederalkenyl ist, $R_2$ Wasserstoff, Niederalkyl, Niederalkenyl, Niederalkanoyl, gegebenenfalls durch Niederalkyl, Niederalkoxy und/oder Halogen substituiertes Benzoyl, Hydroxy, Niederalkoxy, Niederalkenyloxy,Halogen oder Niederalkanoyloxy darstellt, $R_4$ eine Gruppe der Formel $-C(R_6)=C(R_7)-C(=O)-R_8$ bedeutet, in der $R_6$ Wasserstoff, Niederalkyl, Niederalkenyl oder Hydroxyniederalkyl und $R_7$ Wasserstoff,

Niederalkyl oder Niederalkenyl bedeutet, und $R_8$ gemeinsam mit einem der Reste $R_3$ und $R_5$ Oxy bedeutet und der andere der Reste $R_3$ und $R_5$ Wasserstoff oder, im Falle von $R_5$, auch Hydroxy bedeutet, und Salze herstellt.

5. Verfahren nach einem der Ansprüche 1 bis 3, dadurch gekennzeichnet, dass man Verbindungen der Formel I, worin R Carboxy Niederalkoxycarbonyl oder Niederalkanoyloxyniederalkoxycarbonyl bedeutet, $R_1$ Wasserstoff oder Niederalkyl darstellt, $R_2$ Wasserstoff, Niederalkyl, Niederalkenyl, Hydroxy, Niederalkoxy, Niederalkanoyloxy oder Halogen bedeutet, $R_4$ eine Gruppe der Formel $-C(R_6)=C(R_7)-C(=O)-R_8$ darstellt, in der $R_6$ und $R_7$ unabhängig voneinander Wasserstoff oder Niederalkyl bedeuten und $R_8$ gemeinsam mit einem der Reste $R_3$ und $R_5$ Oxy bedeutet und der andere der Reste $R_3$ und $R_5$ Wasserstoff ist, und Salze herstellt.

6. Verfahren nach einem der Ansprüche 1 bis 3, dadurch gekennzeichnet, dass man Verbindungen der Formel I, worin R Carboxy oder Niederalkoxycarbonyl mit bis und mit 4 C-Atomen im Niederalkylteil bedeutet, $R_1$ für Wasserstoff steht, $R_2$ Wasserstoff, Niederalkyl mit bis und mit 4 C-Atomen oder Niederalkenyl mit bis und mit 4 C-Atomen bedeutet, $R_4$ eine Gruppe der Formel $-C(R_6)=C(R_7)-C(=O)-R_8$ darstellt, in der $R_6$ Niederalkyl mit bis und mit 4 C-Atomen darstellt, $R_7$ Wasserstoff bedeutet und einer der Reste $R_3$ und $R_5$ gemeinsam mit $R_8$ Oxy darstellt und der andere Wasserstoff bedeutet, und Salze herstellt.

7. Verfahren nach einem der Ansprüche 1 bis 3, dadurch gekennzeichnet, dass man Verbindungen der Formel I, worin R Carboxy bedeutet, $R_1$ für Wasserstoff steht, $R_2$ Niederalkyl mit bis und mit 4 C-Atomen oder Niederalkenyl mit bis und mit 4 C-Atomen bedeutet, $R_4$ eine Gruppe der Formel $-C(R_6)=C(R_7)-C(=O)-R_8$ darstellt,

in der $R_6$ Niederalkyl mit bis und mit 4 C-Atomen bedeutet, $R_7$ Wasserstoff bedeutet und einer der Reste $R_3$ und $R_5$ gemeinsam mit $R_8$ Oxy darstellt und der andere Wasserstoff bedeutet, und Salze herstellt.

8. Verfahren nach einem der Ansprüche 1 bis 3, dadurch gekennzeichnet, dass man 4-Methyl-2,6-dioxo-2H,6H-benzo[1,2-b:5,4-b']-dipyran-8-carbonsäure oder ein Salz davon herstellt.

9. Verfahren nach einem der Ansprüche 1 bis 3, dadurch gekennzeichnet, dass man 4-Methyl-10-propyl-2,6-dioxo-2H,6H-benzo[1,2-b:5,4-b']-dipyran-8-carbonsäure oder ein Salz davon herstellt.

10. Verfahren nach einem der Ansprüche 1 bis 3, dadurch gekennzeichnet, dass man 4-Methyl-6-propyl-2,10-dioxo-2H,10H-benzo-[1,2-b:3,4-b']-dipyran-8-carbonsäure oder ein Salz davon herstellt.

11. Verfahren nach einem der Ansprüche 1 bis 3., dadurch gekennzeichnet, dass man 4-Methyl-10-allyl-2,6-dioxo-2H,6H-benzo[1,2-b:5,4-b']-dipyran-8-carbonsäure oder ein Salz davon herstellt.

12. Verfahren nach einem der Ansprüche 1 bis 3, dadurch gekennzeichnet, dass man 6-Allyl-4-methyl-2,10-dioxo-2H,10H-benzo[1,2-b:3,4-b']-dipyran-8-carbonsäure oder ein Salz davon herstellt.

13. Verfahren zur Herstellung pharmazeutischer Präparate, dadurch gekennzeichnet, dass man eine Verbindung gemäss einem der Ansprüche 1 und 3 bis 12, gegebenenfalls unter Beimischung von üblichen Hilfs- und Trägerstoffen, zu pharmazeutische Präparaten herstellt.

FO 7.4 DH/rz*

0108036

Europäisches Patentamt

# EUROPÄISCHER RECHERCHENBERICHT

Nummer der Anmeldung

EP 83 81 0429

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| --- | --- | --- | --- |
| A | CHEMICAL ABSTRACTS, Band 70, 1969, Seite 115, Nr. 75488u, und Eighth Collective Index, 3. Spalte, Columbus, Ohio, US L.M. FILIPPOVA: "Decrease in the mutagenic activity of chemical compounds, produced by nitro substitution" & MECH. MUTAT. INDUCING FACTORS, PROC. SYMP. 1965 (Pub. 1966), 295-300<br>--- | 1 | C 07 D 493/04<br>A 61 K 31/37 //<br>(C 07 D 493/04<br>C 07 D 311/00<br>C 07 D 311/00 ) |
| A | GB-A-2 009 157 (FISONS)<br>* Seiten 1-5 *<br>----- | 1,12, 14-17 | |
| | | | RECHERCHIERTE SACHGEBIETE (Int. Cl. 3) |
| | | | C 07 D 493/00 |

Der vorliegende Recherchenbericht wurde für alle Patentansprüche erstellt.

| Recherchenort | Abschlußdatum der Recherche | Prüfer |
| --- | --- | --- |
| DEN HAAG | 10-01-1984 | FRANCOIS J.C.L. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82